# EUROPEAN PATENT APPLICATION

(11) **EP 3 597 769 A1**
(43) Date of publication of application: **22.01.2020**
(21) Application number: 18744200.9
(22) Date of filing: 25.01.2018
(51) Int. Cl.: C12Q 1/68

(54) **SECOND GENERATION SEQUENCING-BASED METHOD FOR SIMULTANEOUSLY DETECTING MICROSATELLITE LOCUS STABILITY AND GENOMIC CHANGES**

(30) Priority: 25.01.2017 CN 201710061152
(71) Applicant: Guangzhou Burning Rock DX Co., Ltd., Guangzhou, Guangdong 510300 (CN)
(72) Inventor: ZHANG, Zhihong, Guangzhou Guangdong 510300 (CN); HAN, Yusheng, Guangzhou Guangdong 510300 (CN); CHUAI, Shaokun, Guangzhou Guangdong 510300 (CN); LIU, Chenglin, Guangzhou Guangdong 510300 (CN); ZHANG, Zhou, Guangzhou Guangdong 510300 (CN); DENG, Wanglong, Guangzhou Guangdong 510300 (CN); LI, Bingsi, Guangzhou Guangdong 510300 (CN); LUO, Fang, Guangzhou Guangdong 510300 (CN); LIU, Jing, Guangzhou Guangdong 510300 (CN); HAN-ZHANG, Han, Guangzhou Guangdong 510300 (CN)
(74) Representative: Zacco GmbH
(86) International application number: PCT/CN2018/074092
(87) International publication number: WO 2018/137678

(57) **Abstract**

Provided is a second generation sequencing-based method for simultaneously detecting microsatellite locus stability and genomic changes (prettyMSI), especially an application of the detection method in assisting in the diagnosis of patients with colorectal cancer and a corresponding kit. Microsatellite loci are selected from the 22 microsatellite loci as shown in table 1, or any combination of 15, 16, 17, 18, 19, 20, and 21 microsatellite loci of the 22 microsatellite loci.

## Description

### Technical Field

The present invention relates to a method for simultaneous detection of microsatellite stability and disease-related genes based on Next-generation sequencing. The invention also relates to the application of the detection method in the auxiliary diagnosis, prognosis evaluation, or selection of treatment regimen for colorectal cancer patients, and the application of the corresponding kit and detection reagent in the preparation of kit.

### Background Art

The incidence of colorectal cancer (CRC) is the third highest one among various cancers in China, accounting for the fifth leading cause of cancer death. The 5-year survival rate after radical resection is about 50% and postoperative recurrence and metastasis are important causes of death. Patient diagnosed with early colorectal cancer can usually be treated by surgical removal. Once colorectal cancer transfers, there are not many methods of treatment, and the five-year survival rate is not satisfactory. The study finds that genomic instability is closely related to the pathogenesis of colorectal cancer. The genomic instability includes chromosomal instability (CI) and microsatellite instability (MSI). About 80-85% of the CRC manifests as CIN, including Familial Adenomatous Polyposis (FAP) (APC germline mutation) and sporadic CRC (APC, P53, DCC, KRAS and other gene mutations); another 15-20% of CRC manifests as MSI, including Hereditary Nonpolyposis Colorectal Cancer (HNPCC, also known as Lynch syndrome) (mismatch repair gene germline mutation) and sporadic MSI (+) CRC (mismatch repair gene MLH1 gene promoter methylation).

A microsatellite is a repetitive DNA short sequence or single nucleotide region contained in a gene. In tumor cells, when DNA methylation or gene mutations cause mismatch repair gene deletion, micro satellite repeat sequence mismatch (microsatellite mutation) can be caused, leading to its sequence shortened or lengthened, thereby resulting in microsatellite instability (MSI). According to the degree of MSI, it can be classified into high microsatellite instability (MSI-H) and low microsatellite instability (MSI-L), and microsatellite stability (MSS) which is called under normal conditions.

A large number of studies have shown that MSI is involved in the development of malignant tumors and is closely related to colorectal cancer, gastric cancer and endometrial cancer. There is MSI phenotype among about 15% of patients with colorectal cancer, and among more than 90% of patients with typical hereditary nonpolyposis colorectal cancer (HNPCC) therein, indicating that MSI can be used as an important marker for detecting whether the patients have HNPCC. Patients with MSI colorectal cancer have a better prognosis, compared with those with MSS (i.e. microsatellite stability) colorectal cancer, whose drug responses are different, suggesting that MSI can be used as an independent predictor of colorectal cancer prognosis. Therefore, MSI detection is of great significance for patients with colorectal cancer.

The latest edition of the 2016 year's National Comprehensive Cancer Network guidelines (NCCN, 2016 Version 2) for colorectal cancer treatment clearly states for the first time that "all patients with a history of colon/rectal cancer should be tested for MMR (mismatch repair) or MSI", because the prognosis for MSI-H (i.e., high microsatellite instability) stage II colorectal cancer patients is good (5y-OS rate for surgery alone is 80%) and the patients cannot benefit from 5FU adjuvant chemotherapy (which is however harmful). And the guidelines recommend for the first time PD-1 monoclonal antibody Pembrolizumab and Nivolumab for the end-line therapy of the mCRC's patients with dMMR/MSI-H molecular phenotype. This fully demonstrates the importance of detecting MMR and MSI in advanced colorectal cancer. At the same time, due to the association of a large number of genes with hereditary colorectal cancer, it is recommended for the patients and their families with a clear family history to employ multi-gene panel sequencing for the first detection.

At present, the MMR gene detection carried out in domestic hospitals is a protein test based on immune histochemical detection, usually detecting MLH1 and MSH2 only, and partially detecting both MSH6 and PMS2, and the positive results thereof is less consistent with the MSI detection results. A few hospitals have carried out MSI state detection by PCR combined with capillary electrophoresis method, and most of them are outsource detection. This method usually selects 5-11 single nucleotide repeat sites with a length of about 25 bp. After PCR operation, the length distribution interval is measured by capillary electrophoresis to determine the microsatellite stability of the sample. This method is the current gold standard detection method, but needs additional samples to be detected, and also needs the patients ' normal tissue as a control for the state determination. And thus the current method is not convenient from an operational point of view.

Therefore, how to establish an efficient and convenient system of MSI detection and to find highly sensitive and specific MSI loci for detecting colorectal cancer has become a research hotspot in recent years. In addition, how to simultaneously detect the state of microsatellite stability and other disease-related genes of the sample is also a problem that needs to be solved.

### Summary of the Invention

The technical problem to be solved by the present invention is to overcome the deficiencies of the prior art, and provide an MSI detection method based on a Next-generation sequencing platform. Based on the detection method, highly sensitive and specific MSI loci used for diagnosing colorectal cancer is obtained. The method is named "pretty MSI (Microsatellite Instability detection based on Peak Ratio Estimation using Tumor Tissue only)", and its corresponding Chinese name is "Method for detecting microsatellite instability based on target peak height ratio in Next-generation sequencing utilizing only tumor tissue". Furthermore, the present invention establishes a method for determining candidate microsatellite loci in a genetic detection that can be used for colorectal cancer prognosis assessment and/or treatment regimen selection. The invention also realizes simultaneous detection of multiple microsatellite loci and multiple disease-related genes in the sample, and can provide more comprehensive conclusions and suggestions on prognosis, treatment, and detection for the samples detected.

The inventors of the present application found that for a microsatellite locus of a normal sample, the sequencing fragments' read covers on one or two types of repeat lengths of the sample genotype with a large probability; while for a microsatellite instable sample, the microsatellite locus causes a large number of repetitive sequences to expand or contract due to the incorrect replication of DNA, so that the covering probability of the sequencing fragments' read on the corresponding genotype of the normal sample was significantly reduced. In the present invention, the genotype corresponding to the normal sample is referred to as the target genotype. Due to the inevitable error rate of sequence capturing and sequencing, the sequencing fragments' reads from different samples have different coverage rate on the target genotype. It has been found in the present invention that the coverage rate of the target genotype by the marker microsatellite locus in the normal sample is sufficiently stable, whereas the coverage rate of the target genotype in the microsatellite instability sample is much smaller than the normal value. Based on this finding, the inventors obtain 22 loci (see Table 1 for details) by screening, and further constructs basic length distribution reference sets for microsatellite state detection, which is consisting of individual coverage ratio vector R of 22 loci and coverage rate NT on target genotype. The reference sets' data is based on a large number of normal tissue samples.

Table 1 Information of the detection marker of microsatellite locus related to colorectal cancer diagnosis

| No. | Chromosome | Position | Repeat number | Nucleotide | Start sequence | Termination sequence | Name | Average coverage rate (mean) | Standard deviation (sd) |
|---|---|---|---|---|---|---|---|---|---|
| 1 | 1 | 161332091 | 14 | T | ATTCC | GCTTT | MS-BR1 | 0.661 | 0.039 |
| 2 | 2 | 47635523 | 13 | T | TGTAC | AAGGA | MS-BR2 | 0.913 | 0.019 |
| 3 | 2 | 47641559 | 27 | A | CAGGT | GGGTT | MS-BR3* | 0.738 | 0.032 |
| 4 | 2 | 48032740 | 13 | T | TGTGA | AAGGT | MS-BR4 | 0.974 | 0.011 |
| 5 | 2 | 48033890 | 18 | T | AAAAC | AATTT | MS-BR5 | 0.873 | 0.055 |
| 6 | 2 | 95849361 | 23 | T | TCCTA | GTGAG | MS-BR6* | 0.747 | 0.058 |
| 7 | 4 | 55598211 | 25 | T | TTTGA | GAGAA | MS-BR7* | 0.440 | 0.037 |
| 8 | 7 | 6037057 | 17 | A | AACTG | TTCAC | MS-BR8 | 0.895 | 0.042 |
| 9 | 7 | 116381121 | 16 | T | TGGTG | GGTTT | MS-BR9 | 0.794 | 0.052 |
| 10 | 7 | 116409675 | 15 | T | CAACC | CCTTT | MS-BR10 | 0.875 | 0.034 |
| 11 | 11 | 108114661 | 15 | T | AATAA | AAGAA | MS-BR11 | 0.750 | 0.043 |
| 12 | 11 | 108121410 | 15 | T | TATCC | AGGCT | MS-BR12 | 0.784 | 0.064 |
| 13 | 11 | 108141955 | 15 | T | TGAAC | ACCAC | MS-BR13 | 0.638 | 0.031 |
| 14 | 11 | 108188266 | 13 | T | CTTGA | GCCTC | MS-BR14 | 0.853 | 0.057 |
| 15 | 11 | 108195976 | 19 | T | CATAG | CATTT | MS-BR15 | 0.733 | 0.072 |
| 16 | 11 | 125490765 | 21 | T | GAAGA | AATAT | MS-BR16* | 0.832 | 0.046 |
| 17 | 12 | 133237753 | 14 | A | ACCTG | GGCAA | MS-BR17 | 0.724 | 0.038 |
| 18 | 13 | 32905219 | 12 | T | TTTGA | GAGGT | MS-BR18 | 0.913 | 0.021 |
| 19 | 13 | 32907535 | 11 | T | CTGTC | GTAAA | MS-BR19 | 0.913 | 0.019 |
| 20 | 14 | 23652346 | 21 | A | TTGCT | GGCCA | MS-BR20* | 0.792 | 0.089 |
| 21 | 15 | 91303325 | 12 | T | AAGAC | CCCTC | MS-BR21 | 0.816 | 0.031 |
| 22 | 18 | 48584855 | 16 | T | GGCTA | GGTAG | MS-BR22 | 0.776 | 0.059 |

| | | | | | | | | | |
|---|---|---|---|---|---|---|---|---|---|
| Note: * represents the five marker that need to be detected for microsatellite stability using NGS and PCR technology, i.e., BAT-26, NR-24, BAT-25, NR-22 and NR-21, respectively. The mean and "***sd***" listed in the table are the mean and variance of the normal range obtained by the length distribution reference sets of each point. If the target genotype coverage is less than ***mean-3sd**,* the position is determined as the target MSI locus, wherein the calculation of ***mean-3sd*** is suitable for 22 loci. | | | | | | | | | |

Based on the characteristics of Next-generation sequencing, the present invention has developed a novel method for detection of microsatellites stability (pretty MSI). The detection method of the present invention has the correct matching rate compared with the gold standard method for MSI detection, which is based on the PCR and electrophoresis method. And the present detection method is faster and cheaper. At the same time, according to the method of the invention, a stable normal sample microsatellite length distribution reference set is constructed, and the detection method can be completed without providing a matching normal sample, which is of great significance in practical applications.

Furthermore, in addition to basing on the technology of Next-generation sequencing, the method of the present invention can perform multi-sample sequence analysis at one time, and can complete detection of microsatellite stability while detecting genomic changes such as gene mutations and chromosomal variations. The detection can be implemented without additional operations. The method of the present invention greatly saves detection time and costs. The multi-gene panel of the present invention further includes detecting genes associated with colorectal cancer treatment and prognosis as defined in the NCCN treatment guidelines, genes for predisposition of colorectal cancer as defined in the NCCN genetic screening guidelines, and others gastrointestinal tumor-associated exon gene. Specifically, the multi-gene panel comprises the following 36 genes: BRAF, HRAS, KRAS, NRAS, PTCH1, APCBLM, BMPR1A, CHEK2, EpCAM, GREM1, MLH1, MSH2, MSH6, MUTYH, PMS2, POLD1, POLE, PTEN, SMAD4, STK11, TP53, AKT1, ATM, BRCA1, BRCA2, CDH1, EGFR, ERBB2, KIT, MET, PDGFRA, PIK3CA, SDHB, SDHC, SDHD.

The technical solution of the present invention specifically includes the following contents:

In one aspect, the invention relates to a biomarker panel comprising 22 microsatellite loci as shown in Table 1, or comprising a combination of 15, 16, 17, 18, 19, 20, or 21 microsatellite loci as shown in Table 1. Preferably, the biomarker panel is a combination of 22 microsatellite loci as shown in Table 1.

In another aspect, the invention relates to a biomarker panel comprising a combination of microsatellite loci and one or more genes, wherein the combination of microsatellite loci includes 22 microsatellite loci as shown in Table 1 or a combination of 15, 16, 17, 18, 19, 20, or 21 microsatellite loci as shown in Table 1, and wherein one or more genes are selected form the following 36 genes: BRAF, HRAS, KRAS, NRAS, PTCH1, APCBLM, BMPR1A, CHEK2, EpCAM, GREM1, MLH1, MSH2, MSH6, MUTYH, PMS2, POLD1, POLE, PTEN, SMAD4, STK11, TP53, AKT1, ATM, BRCA1, BRCA2, CDH1, EGFR, ERBB2, KIT, MET, PDGFRA, PIK3CA, SDHB, SDHC, SDHD. Preferably, the biomarkers panel comprises 22 microsatellite loci as shown in Table 1, and/or one or more of the 36 genes.

In another aspect, the invention relates to a kit that can be used for genetic detection for prognostic evaluation and/or treatment regimen selection for stage II colorectal cancer, and for diagnosis, prognosis assessment, and/or treatment regimen selection of Lynch syndrome (HNPCC). The kit includes the detection reagents for detecting the biomarker panel.

In another aspect, the present invention relates to a kit. The kit can be used for simultaneously performing genetic detections to assess prognosis and/or select treatment regimen for colorectal cancer, assess genetic susceptibility to colorectal cancer, and assess genetic susceptibility to gastrointestinal cancer. The kit comprises a reagent for detecting the biomarker panel as described above. Preferably, the genetic detection comprises detection the genes of familial adenomatous polyposis, sporadic CRC, Lynch syndrome, and/or sporadic MSI+CRC. Preferably, the detection reagent is a reagent for performing Next-generation sequencing (NGS).

In another aspect, the invention relates to use of a reagent detecting the biomarkers panel in the preparation of a kit, wherein the kit is used for genetic detections to assess prognosis and/or select treatment regimen for colorectal cancer, assess genetic susceptibility to colorectal cancer, and assess genetic susceptibility to gastrointestinal cancer. Preferably, the reagent is a reagent for performing Next-generation sequencing (NGS). More preferably, the genetic detection is used to assess the prognosis of Lynch syndrome (HNPCC) and/or to select a treatment regimen.

In another aspect, the invention relates to a method for determining candidate microsatellite loci in a genetic detection capable of being used for colorectal cancer prognosis assessment and/or treatment regimen selection. The method is based on Next-generation sequencing (NGS), and the method is comprised of the steps as follows:
(1) Simultaneous multi-gene targeted capture detection of multiple microsatellite loci in multiple normal tissue samples based on Next-generation sequencing;
(2) For any one of the microsatellite loci ***i***, the number of sequencing fragments of different lengths the reads of which cover target genotypes corresponding to the loci are counted based on the NGS data, and the target genotype is the genotype of the microsatellite locus in normal tissue samples;
(3) calculating the coverage rate of the target genotypes corresponding to the microsatellite loci according to the number of the sequencing fragments' read and constructing a standard length distribution reference set of the microsatellite loci, thereby calculating the average coverage mean(NTᵢ) and standard deviation sd(NTᵢ) of one or two length types covering the most, wherein if the number of reads of sequencing fragments covering the second largest number is less than 75% of that of the length types covering the most, only the length types covering the most is considered; and calculating the average coverage and standard deviation; if the number of reads of sequencing fragments covering the second largest number is greater than 75% of that of the length types covering the most, the two types of lengths that cover the most are considered, with the average at this time being the sum of the average coverage of the two length types
(4) For colorectal cancer samples, the coverage rate of the target genotype determined is also calculated according to the above steps. If the coverage rate is <mean(NTᵢ)-3sd(NTᵢ), the microsatellite locus is unstable microsatellite locus, which is determined to be a candidate microsatellite locus in a genetic detection, wherein said genetic detection can be used for prognosis assessment and/or treatment regimen selection of colorectal cancer.

Preferably, the microsatellite loci determined by the above method comprises 22 microsatellite loci as described in Table 1 or a combination of 15, 16, 17, 18, 19, 20, 21 loci therein. Preferably, the genetic detection for the colorectal cancer prognosis assessment and/or treatment regimen selection is one for Lynch syndrome (HNPCC) prognostic assessment and/or treatment regimen selection.

In another aspect, the invention relates to a method for determining a stability state of a microsatellite locus in a colorectal cancer sample based on next generation high throughput sequencing, comprising the steps of:
(1) Detecting a multi-genes panel, i.e., detecting a combination of the microsatellite loci based on Next-generation sequencing;
(2) Counting the number of sequencing fragments' read covering different lengths repeats of the loci based on the Next-generation sequencing data;
(3) Calculating coverage rate and constructing a standard length distribution reference set of microsatellite loci through its distribution in a large number of normal samples;
(4) For each microsatellite locus of the sample, the number of the sequencing fragments' read of different lengths is counted by step (2), and the coverage rate of the target genotype is calculated according to step (3);
(5) Comparing the calculated coverage rate with the standard reference set, determining the stability state of the microsatellite loci, and judging the stability state of the microsatellite loci of the sample according to the proportion of the unstable microsatellite loci.

Preferably, in the detection method described above, the detected microsatellite loci comprise 22 microsatellite loci listed in Table 1. In the detection method described above, the judgment in the step (5) is: if the percentage of the number of unstable microsatellite loci is >40%, the sample is determined to be a high MSI; if the percentage is 15%-40%, the sample is determined to be a low MSI sample; if the percentage is <15%, the sample is determined to be an MSS sample.

Preferably, the above method comprises the steps of: (1) simultaneously performing a multi-gene targeted capture detection of a plurality of microsatellite loci in the sample based on Next-generation sequencing, the plurality of microsatellite loci comprising 22 microsatellite loci as shown in Table 1 or a combination of 15, 16, 17, 18, 19, 20, 21 loci therein;
(2)Among the plurality of microsatellite loci, the number of microsatellite loci in which the number of the sequencing fragments' read covering the target genotype corresponding to the microsatellite loci exceeds 10 is n, and the target genotype is the genotype of microsatellite loci in normal tissue samples, n≥15. For any microsatellite locus in n, it can be determined by the above method whether the coverage rate Tᵢⱼ< mean (NTᵢ)-3sd (NTᵢ) is satisfied. When the microsatellite loci are 22 microsatellite loci in Table 1, they can be directly calculated according to the mean (NTᵢ) and sd (NTᵢ) of Table 1;
(3) For the plurality of microsatellite loci, if the number of unstable microsatellite loci is >40%*n, the sample is determined to be a high MSI sample; if the number is 15%-40 %*n, the sample is determined to be a low MSI sample; if the number of samples is <15%*n, the sample is determined to be an MSS.

Preferably, the plurality of microsatellite loci in the above method are 22 microsatellite loci as shown in Table 1.

Specifically, referring to the statistical results in Table 8 below, among the 65 samples, the number of loci with sufficient coverage for each sample is between 15 and 22. The most common of these is the insufficient coverage of the 5 PCR loci, except that there are 3 loci insufficiently covered randomly between samples. However, as mentioned above, microsatellite loci with more than 10 reads of sequencing fragments covering the target genotype corresponding to the microsatellite locus can be included in the statistics.

In another aspect, the invention relates to a method for simultaneously determining the stability of multiple microsatellite loci and detecting disease-related genes for a colorectal cancer sample. Based on Next-generation sequencing, in addition to detection of microsatellite loci, one or more of the following 36 genes are detected simultaneously: BRAF, HRAS, KRAS, NRAS, PTCH1, APCBLM, BMPR1A, CHEK2, EpCAM, GREM1, MLH1, MSH2, MSH6, MUTYH, PMS2, POLD1, POLE, PTEN, SMAD4, STK11, TP53, AKT1, ATM, BRCA1, BRCA2, CDH1, EGFR, ERBB2, KIT, MET, PDGFRA, PIK3CA, SDHB, SDHC, SDHD, and then the stability state results of the microsatellite loci and the detection results of one or more genes are combined to determine the state of the sample.

In another aspect, the invention relates to a kit for using in the method of any of the above, comprising reagents for detecting microsatellite loci and/or reagents for detecting one or more genes. Preferably, the kit comprises reagents for performing Next-generation sequencing. Preferably, the colorectal cancer is Hereditary Nonpolyposis Colorectal Cancer (HNPCC, also known as Lynch syndrome).

### Description of the Drawings

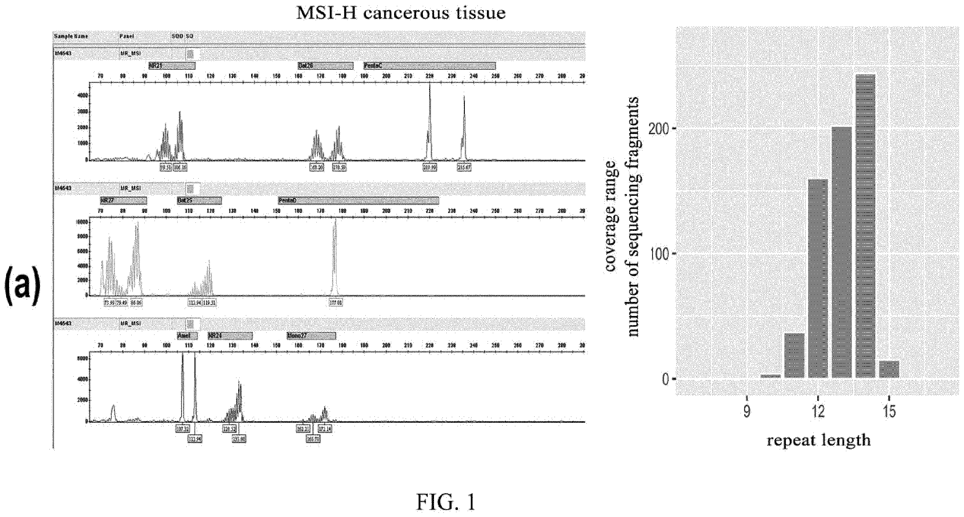

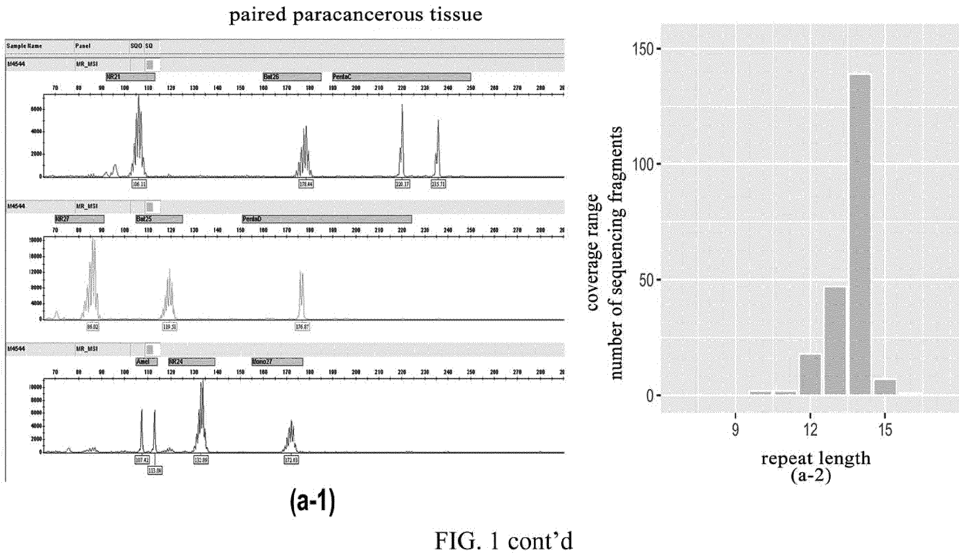

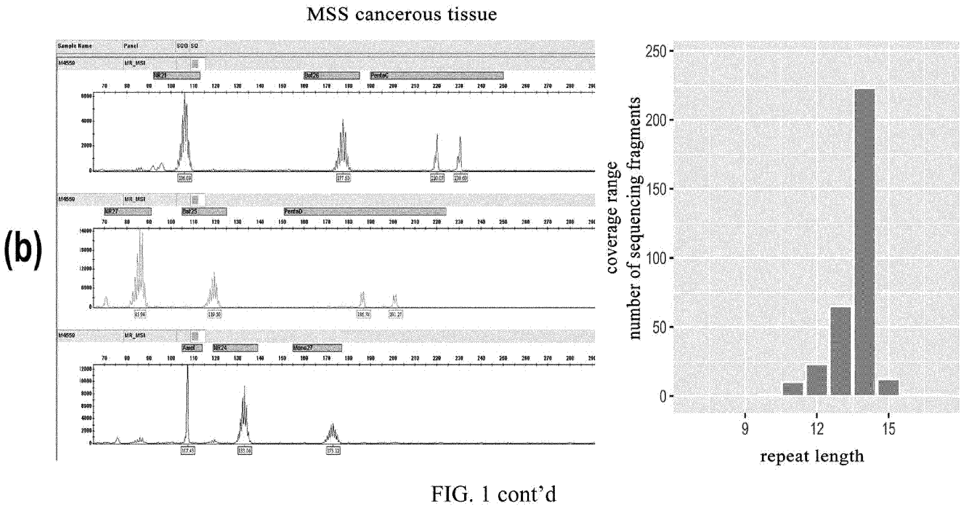

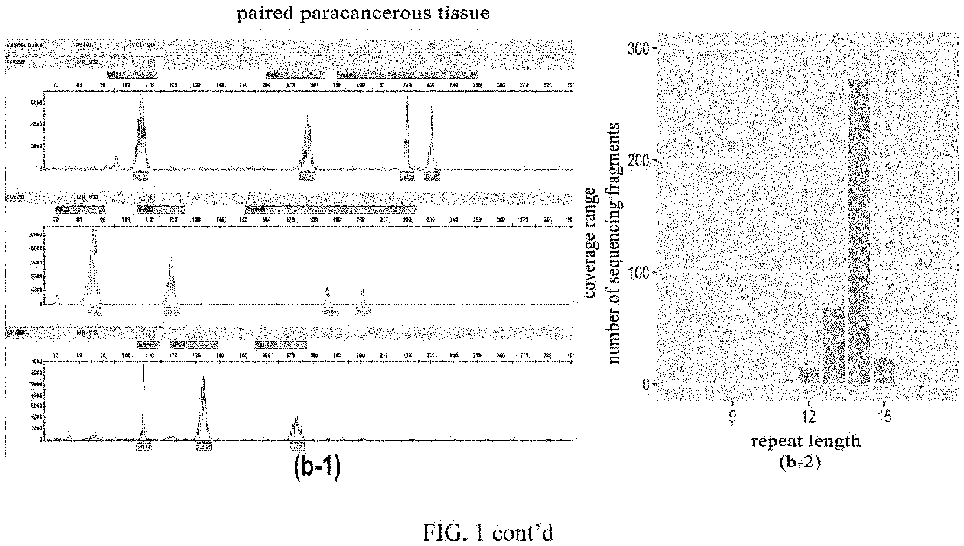

Figure 1 is a graph showing the results of the microsatellite locus MSI-BR1 in Table 1 in different types of samples by using PCR detection or the stability detection. (a) Detection maps of nine marker loci of a MSI-H cancerous tissue (corresponding to RS1607586FFP in Table 7) and its paired paracancerous tissue in PCR detection(a-1); (a-2) Histogram of the number of the sequencing fragments' read covering different repeat lengths of the microsatellite locus MS-BR1. (b) Detection maps of nine marker loci of a MSS cancerous tissue (corresponding to RS1608839FFP in Table 7) and its paired paracancerous tissues in PCR detection (b-1); (b-2) Histogram of the number of the sequencing fragments' read covering different repeat lengths of the microsatellite locus MS-BR1. The abscissa is the repeat length and the ordinate is the coverage range (number of sequencing fragments).
Figure 2 is an example diagram showing the microsatellite locus MSI-BR2 length distribution reference set and a determination criterion.
Figure 3 is histograms of the detection results of 22 microsatellite loci in the cancer tissue sample numbered as RS1611018FFP (rows 1, 3, and 5) and in the corresponding paracancerous tissue samples (rows 2, 4, and 6).
Figure 4 is histograms of the detection results of 22 microsatellite loci in the cancer tissue sample numbered as RS1608823FFP (rows 1, 3, and 5) and in the corresponding paracancerous tissue samples (rows 2, 4, and 6).

### Embodiments

The invention will be further described in detail below in conjunction with specific examples.

### Examples

The experimental samples involved in the examples were all from leading Grade-A Tertiary Hospitals for the diagnosis and treatment of colorectal cancer in China, and all the experimental samples were clearly diagnosed as IHC positive tumors and paracancerous normal tissue samples. The instruments, reagents, kits and analysis software used in the experiment are commercially available.

### Methods and steps

1. Multi-gene panel detection is carried out based on Next-generation sequencing method with the specific steps as follows:
Tumor tissue and paracancerous normal tissue DNA were extracted using QIAamp DNA FFPE tissue kit. Accurate quantification was performed using dsDNA HS assay kits with the Qubit 3.0 fluorometer. The extracted DNA was physically fragmented into 180-250 bp fragments using a sonicator Covaris M220, and then repaired, phosphorylated, added deoxyadenine at the 3' end, and ligated with a linker. The DNA ligated to the amplification linker was then purified using Agencourt AMPure XP paramagnetic beads and pre-amplified using PCR polymerase, and the amplified product was hybridized with Agilent's custom multiplexed biotin-labeled probe set (the multi-gene panel design includes sequences of exons and partial intron regions of 36 genes). After the successfully hybridized fragments were specifically eluted, and amplified by PCR polymerase, quantification and fragment length distribution determination were performed, and Next-generation sequencing was performed using an Illumina sequencer. Tumor tissue's DNA and paracancerous normal tissue's DNA was extracted by using QIAamp DNA FFPE tissue kit. Quantification was performed using a dsDNA HS assay kit with a Qubit 2.0 fluorometer. After disruption of DNA with Covaris M220, end repair, phosphorylation and linker ligation were performed. Fragments of 200-400 bp in length were selected using the Agencourt AMPure XP kit, and hybridized with Agilent multiple capture probes designed according to the exons and partial intron region sequences of the 36 genes included on the multi-gene panel. The successfully hybridized fragments were subjected to magnetic bead purification and PCR amplification and subjected to mass and length measurement, prior to Next-generation sequencing using an Illumina sequencer. The sequenced sequence was aligned to the human genome sequence (version hgl9) using BWA version 0.7.10. And local alignment optimization, variation calling and annotation were performed using GATK 3.2, MuTect and VarScan, respectively. For the variation calling, the VarScanfp filter will remove loci with a depth of 100x or less; for indel and single-locus variations, at least 5 and 8 variable sequencing fragments' read are required, respectively.

2. Microsatellite instability detection method, with the specific steps as follows:
Step 1: Counting the number of the sequencing fragments' reads covering the repeated sequences of different lengths in a locus based on the NGS data. For each microsatellite locus, its position information and the sequence of both ends were first searched in the human genome, and all sequences with intermediate repeat lengths of 1 to L-10 bp connected by the two ends were constructed as a search dictionary, with L being the length of the sequencing fragments reads.
Step 2: Calculating coverage rate and construct a standard reference set for microsatellite loci:
   Calculating the coverage rate of the target genotypes corresponding to the microsatellite loci according to the number of the sequencing fragments' read and constructing a standard length distribution reference set of the microsatellite loci, thereby calculating the average coverage mean (NTᵢ) and standard deviation sd(NTᵢ) of one or two length types covering the most, wherein if the number of reads of sequencing fragments covering the second largest number is less than 75% of that of the length types covering the most, only the length types covering the most is considered; and calculating the average coverage and standard deviation; if the number of reads of sequencing fragments covering the second largest number is greater than 75% of that of the length types covering the most, the two types of lengths that cover the most are considered, with the average at this time being the sum of the average coverage of the two length types.

The microsatellite locus named MSI-BR1 in Table 1 is a representative example illustrating the construction of the standard reference set of the microsatellite micro-locus: MSI-BR01 microsatellite is a single base microsatellite locus on chromosome 1 (14T, T is a repeating base, 14 is the number of repetitions), the sequences at both ends are ATTCC and GCTTT, and the constructed search dictionary comprises ATTCCTGCTTT (repeat length is 1), ATTCCTTGCTTT (repeat length is 2), ATTCCTTTGCTTT (repeat length is 3), and so on. Paired sequencing fragments with at least one end located within 2 kb of the locus were extracted from the sequencing result file of the cancer sample and aligned to sequences in the search dictionary of the locus. The number of the sequencing fragments' reads covering different lengths in the search dictionary was counted and a histogram of the number of the read of the sequencing fragments covering all length types of the locus was constructed.

As shown in Figure 1, the a-2 and b-2 parts are the histograms of the MSI-BR1 high microsatellite instability (MSI-H) and MSI-BR1 microsatellite stability (MSS) loci in cancer and paracancerous samples, respectively. As shown in the figure, the histogram of the MSI-H locus is significantly different between cancer and paracancerous samples. It can be determined from the cancer sample graph in a-2 and the paracancerous sample graph in b-2 that MSI-BR1 is an optional microsatellite locus.

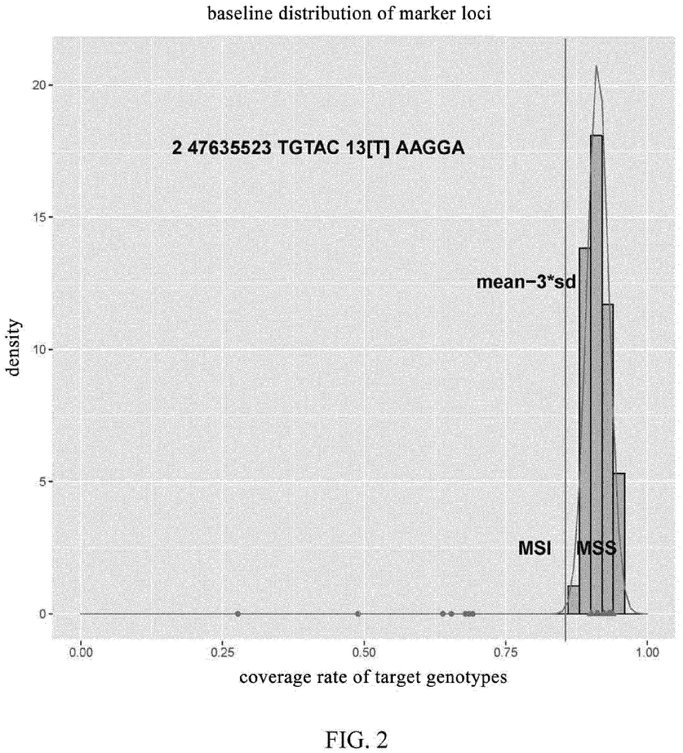

As shown in Figure 2, it is an exemplary diagram of a length distribution reference set the microsatellite locus MSI-BR2 and a determination criterion thereof. The dot to the left of the mean-3sd line on the abscissa shows an example of a MSI-H cancer tissue sample, while the right triangle shows an example of a MSS cancer tissue sample. Wherein the coverage rate of the target genotype conforms to the normal distribution in the reference set, and its average coverage rate is 0.91, and the standard deviation is 0.02, which are obtained according to the above step 2.

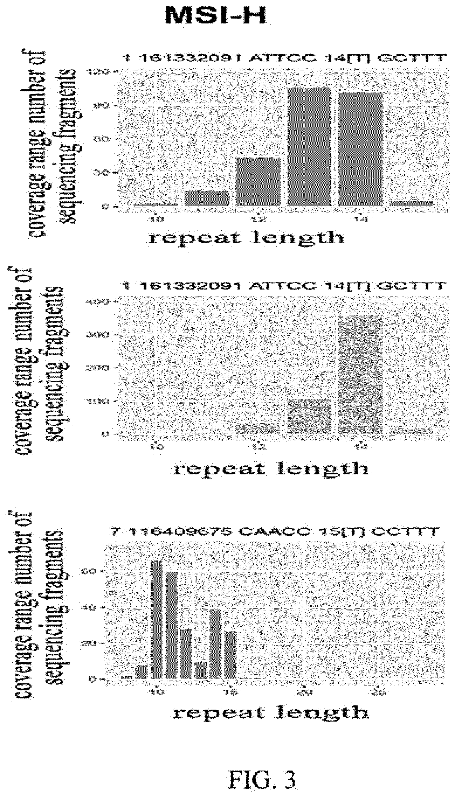

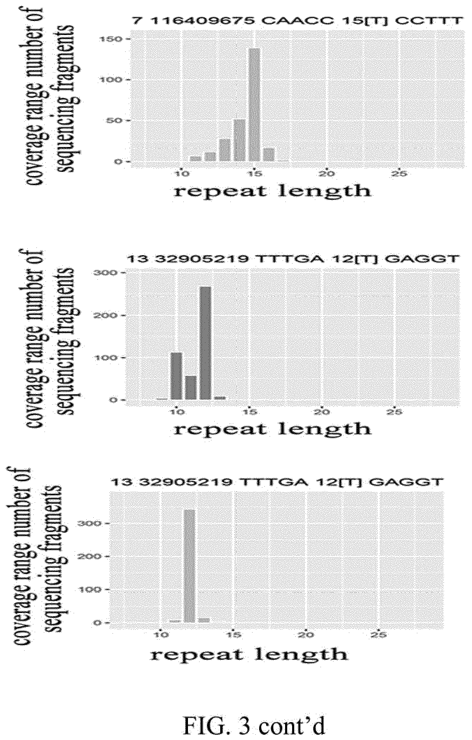

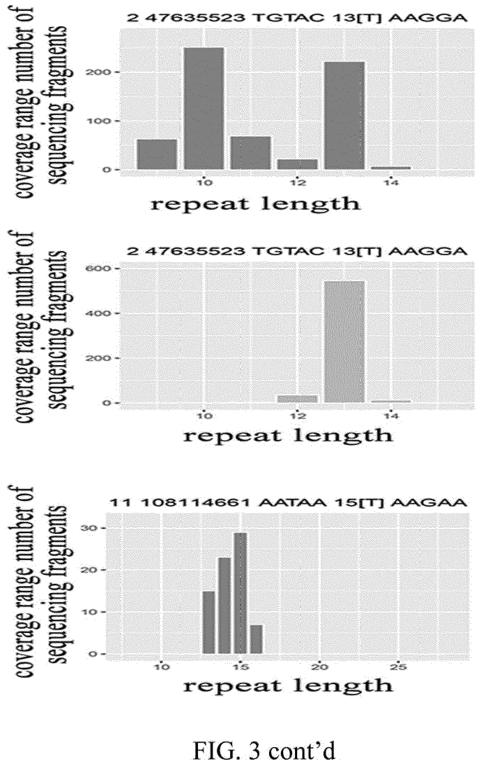

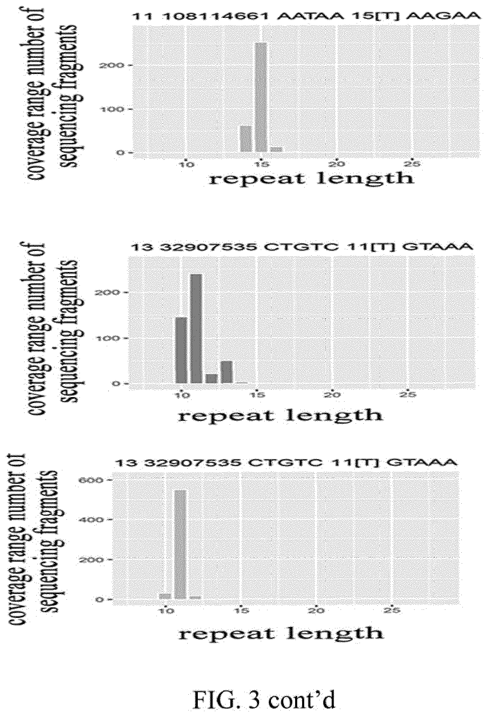

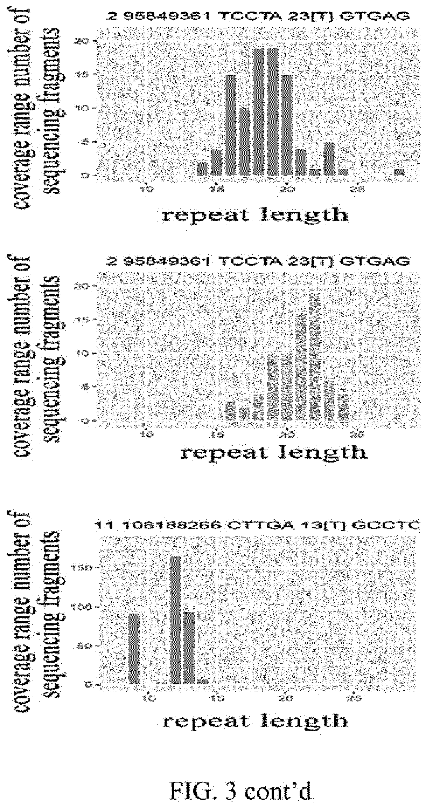

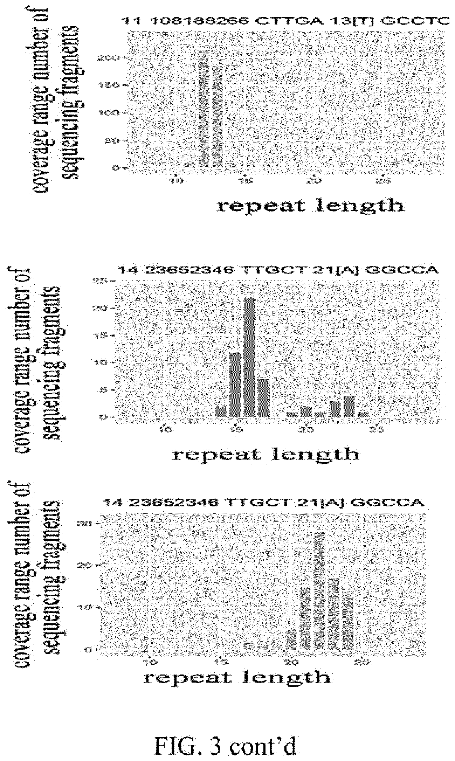

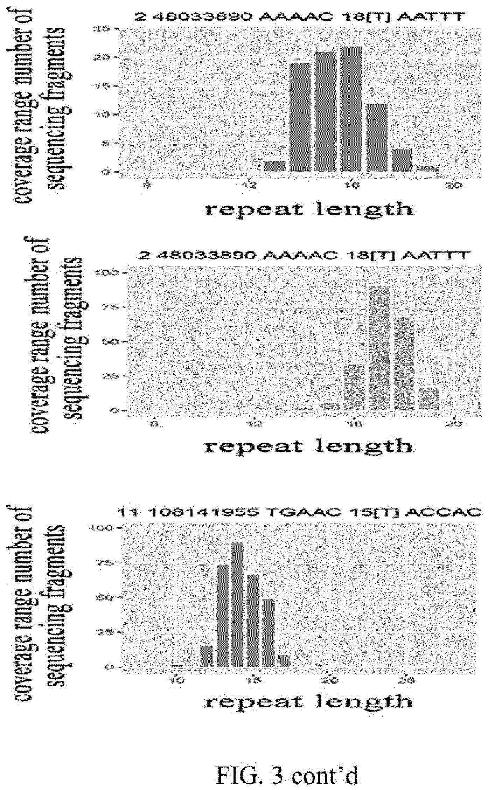

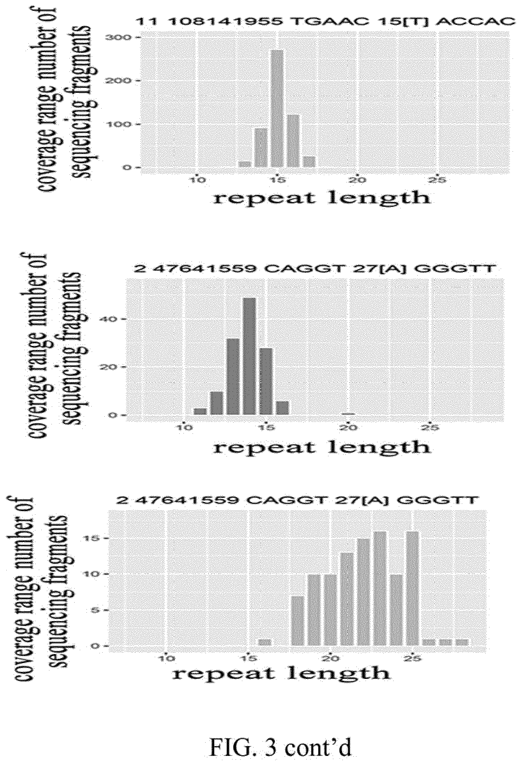

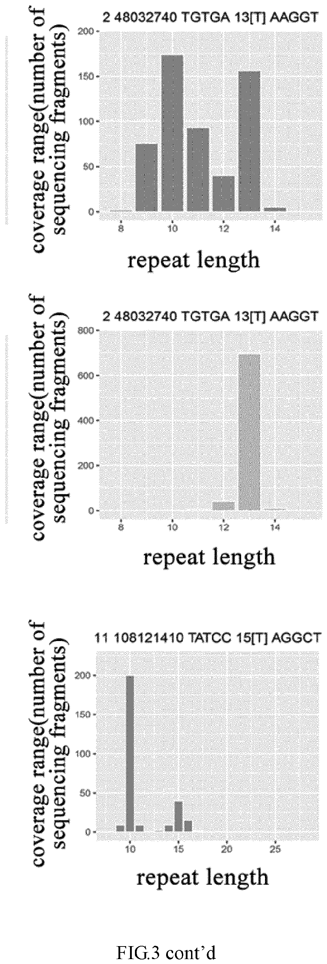

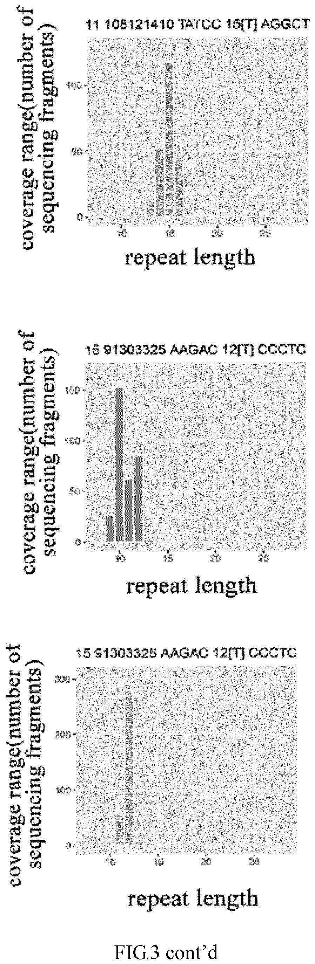

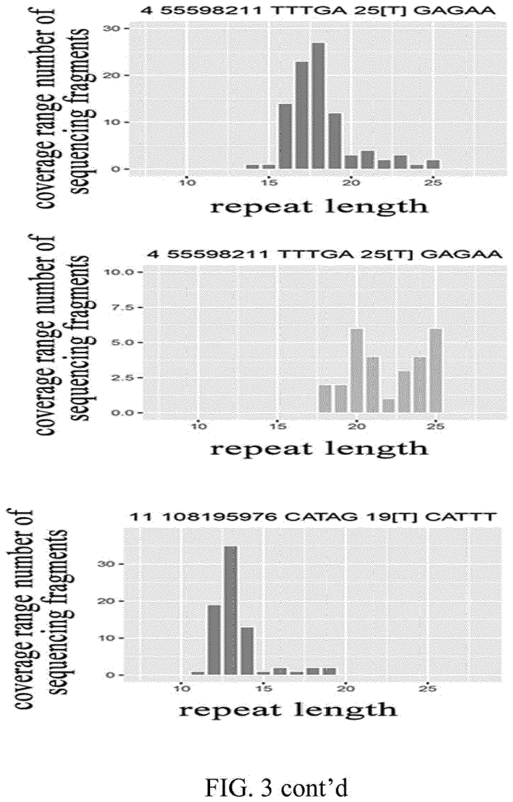

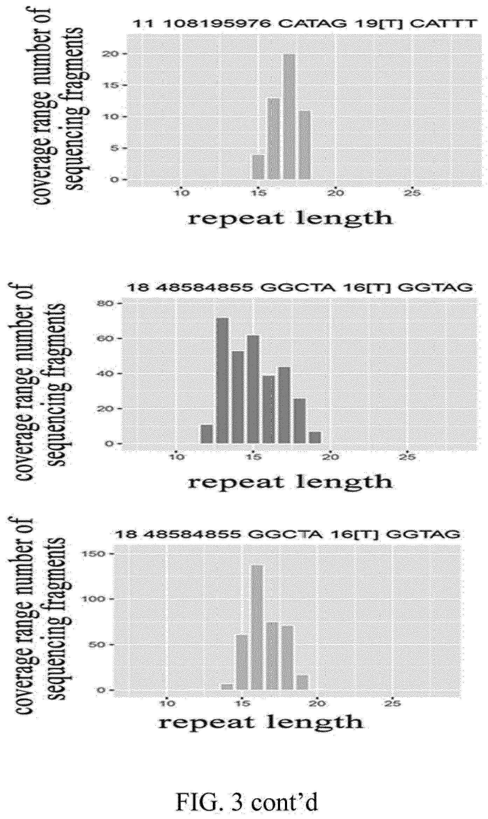

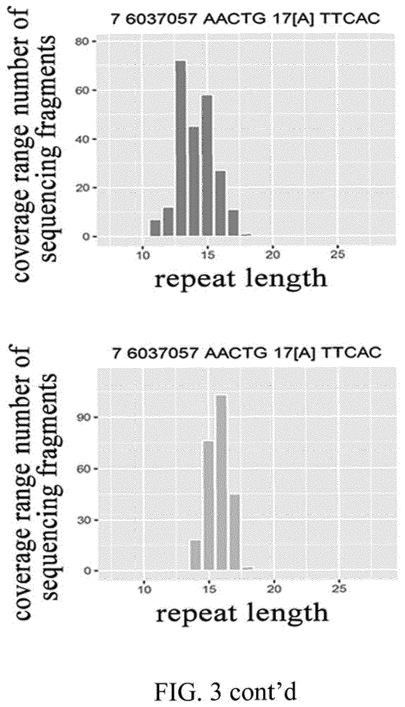

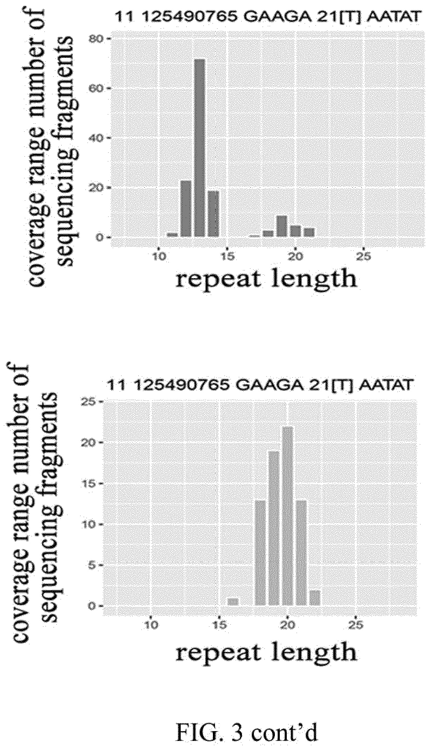

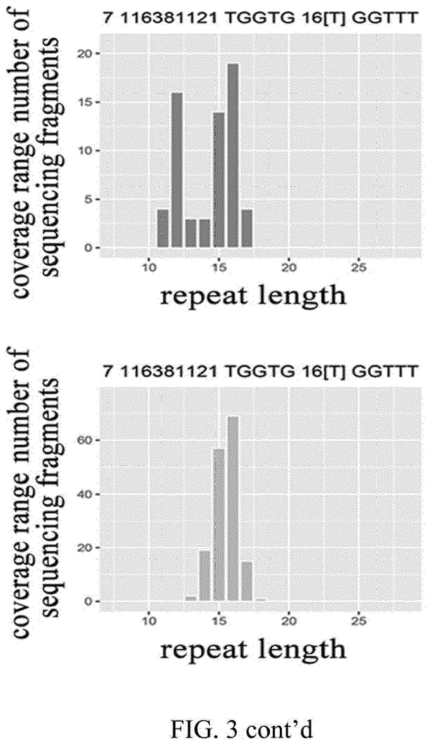

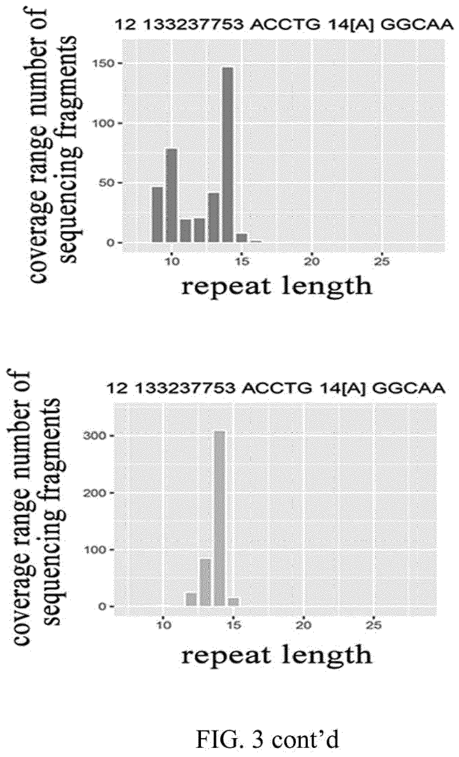

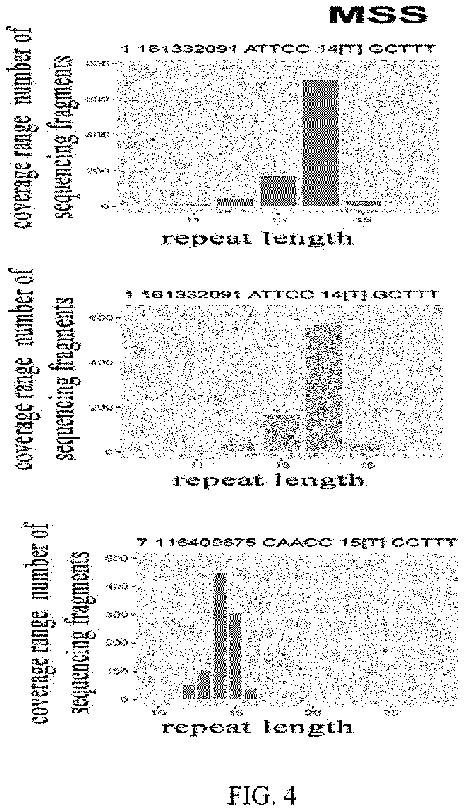

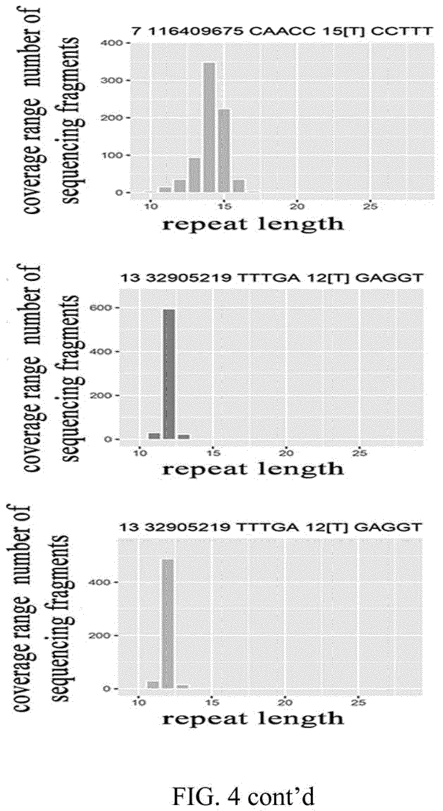

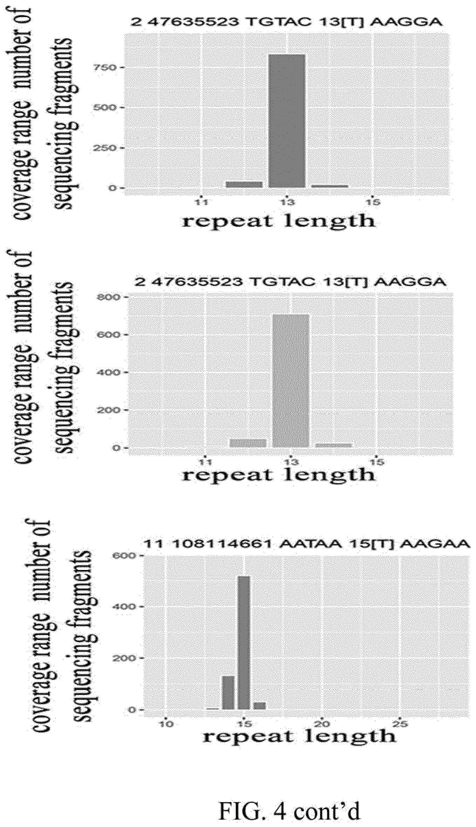

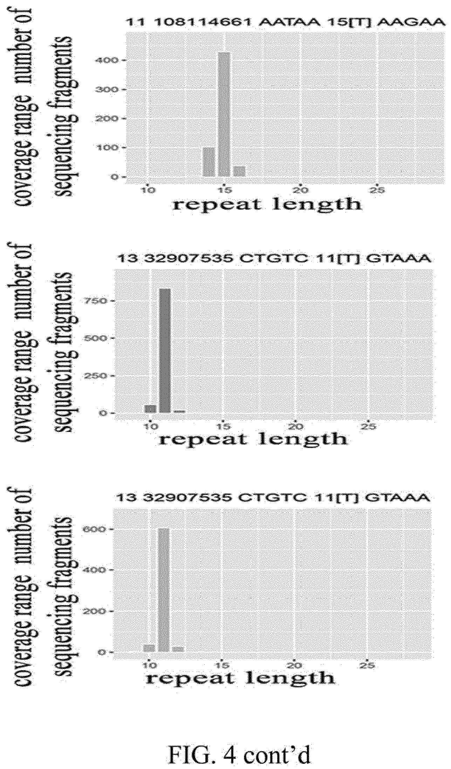

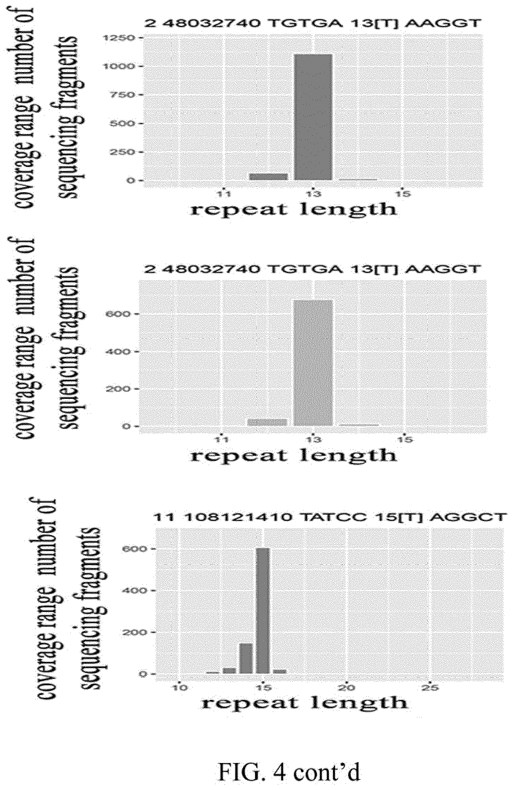

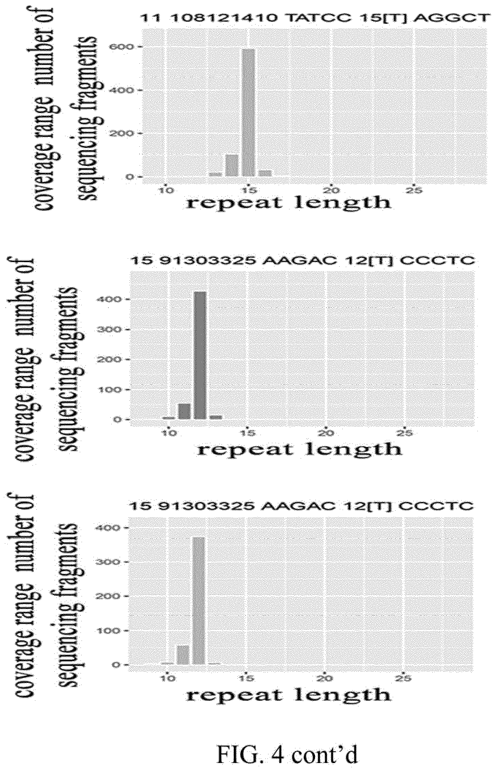

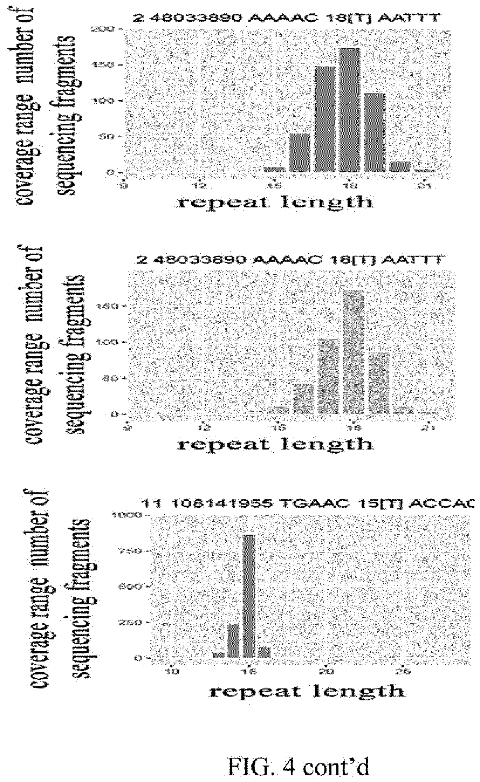

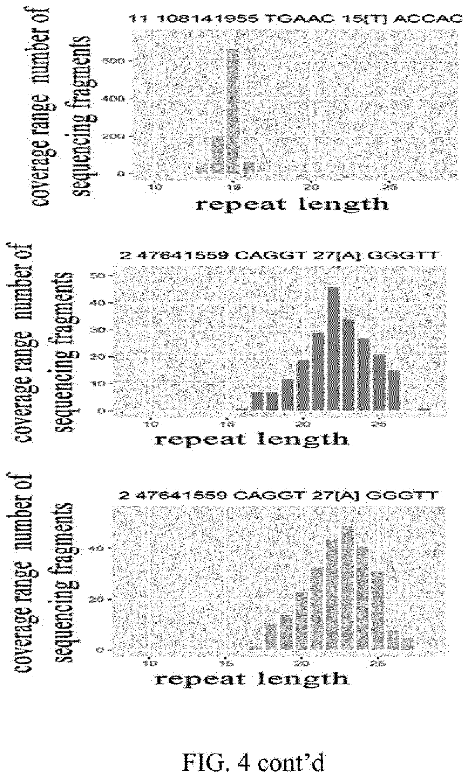

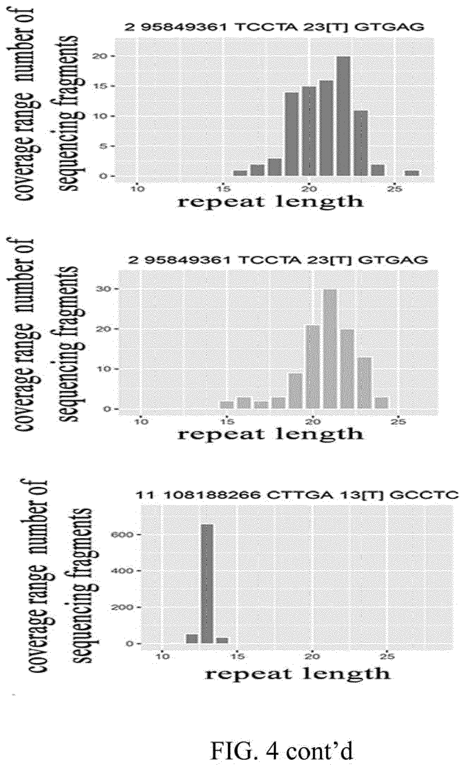

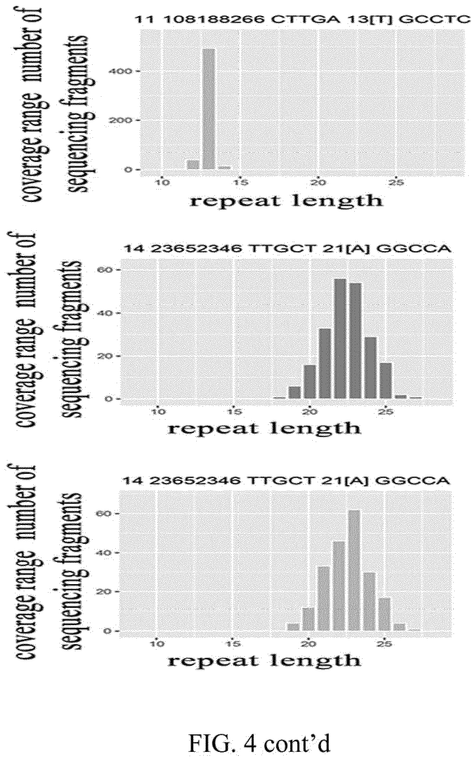

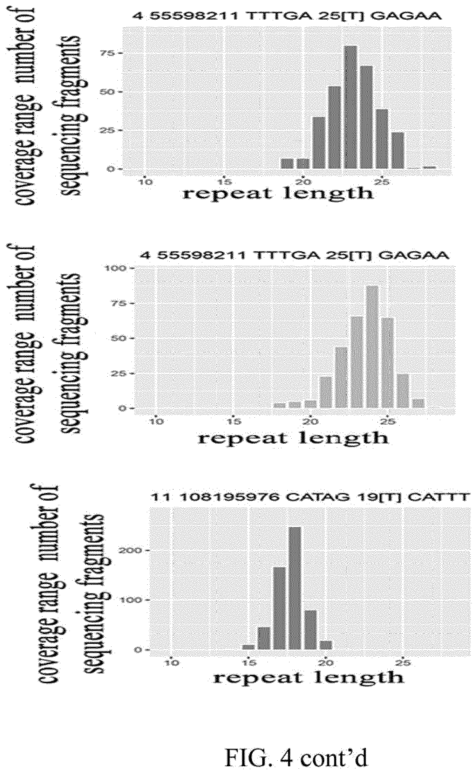

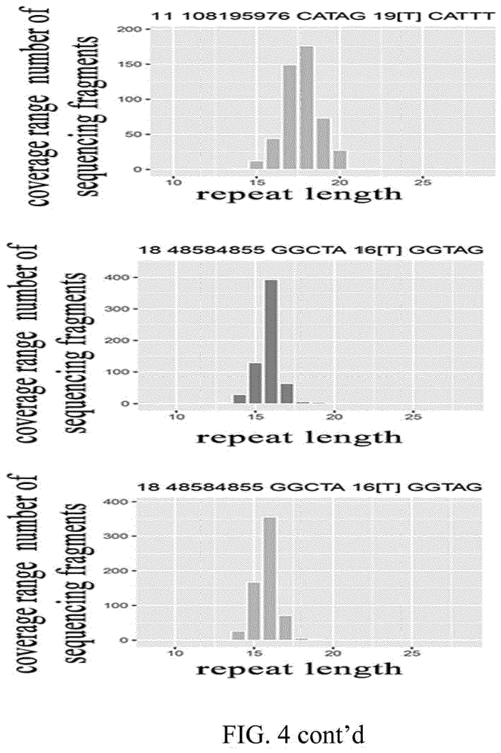

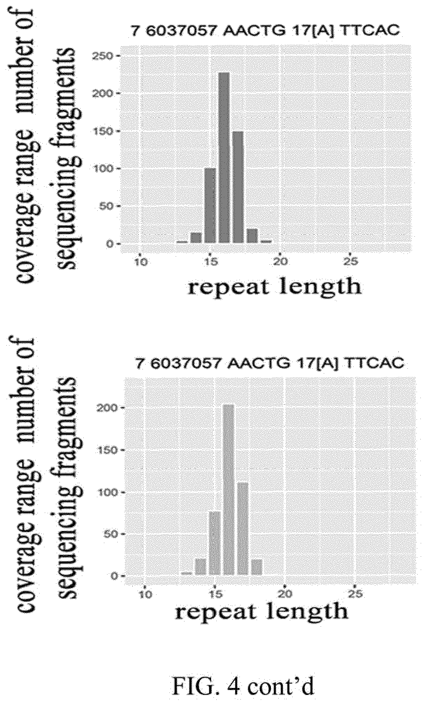

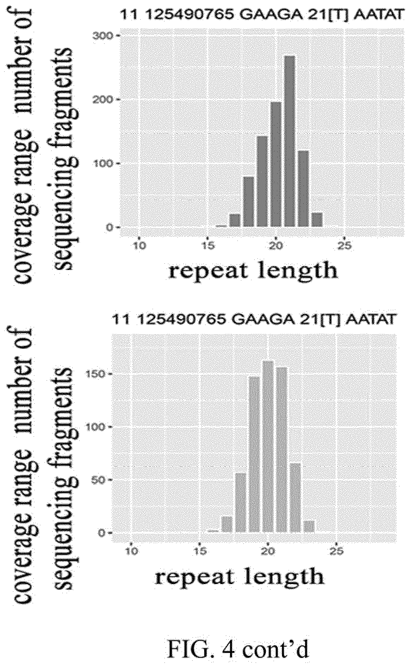

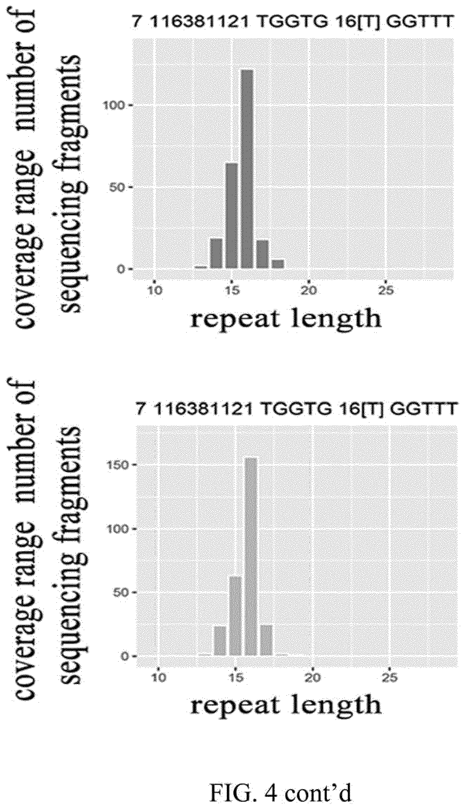

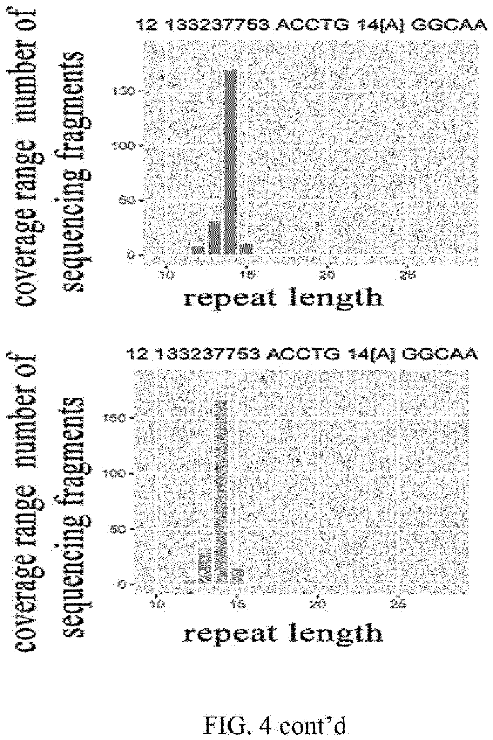

As shown in Figure 3 and Figure 4, it is histograms of the detection results of 22 microsatellite loci in two different numbered cancer tissue samples (rows 1, 3, and 5) and the paracancerous tissue samples (rows 2, 4, and 6). The results are MSI-H and MSS, respectively. The abscissa is the length type of the target genotype, and the ordinate is the number of the sequencing fragments' read of the target genotype.

It should be noted that the upper and lower correspondence of the same locus is only for the convenience of observation. In fact, in the actual detection process, the paracancerous tissue is not required as the control sample. It is only necessary to calculate whether the coverage of the cancer tissue sample meets the coverage rate Tᵢⱼ<mean(NTᵢ)-3sd(NTᵢ), it can be determined whether the microsatellite locus is an unstable microsatellite locus.

Step 3: For each microsatellite locus ***i*** of the cancer sample ***j***, the number of sequencing fragments' read of different lengths is counted by step 1, and the coverage rate (Tᵢⱼ) of the target genotype calculate according to step 2.

Among the plurality of microsatellite loci, the number of microsatellite loci in which the number of the sequencing fragments' read covering the target genotype corresponding to the microsatellite loci exceeds 10 is n, and the target genotype is the genotype of microsatellite loci in normal tissue samples, n≥15. For any microsatellite locus in n, it can be determined by the above method whether the coverage rate Tᵢⱼ< mean (NTᵢ)-3sd (NTᵢ) is satisfied. When the microsatellite loci are 22 microsatellite loci in Table 1, they can be directly calculated according to the mean (NTᵢ) and sd (NTᵢ) of Table 1.

Step 4: The coverage rate of the microsatellite loci listed in Table 1 is detected in turn, and compared with their corresponding standard reference sets, and the stability of the microsatellite loci is determined according to step 3. For a cancer sample, the status of the microsatellite loci listed in Table 1 is checked. If the number of unstable microsatellite loci is >40%*n, the sample is determined to be high MSI; if the number is 15%-40%*n, the sample is determined to be a low MSI sample; if the number of samples is <15%*n, the sample is determined to be an MSS sample.

In addition, for the genotypes that are not present in the reference sets in the sample (sequence fragments' read heavily cover on the new length type), for the loci determined to be microsatellite instability and for the two most covered length type (if the second largest number of sequencing fragments' read is less than 75% of that of the highest peak, only the length type corresponding to the highest peak is considered), if the length is less than 2 bp from the length of the human genome reference sequence, the length sequence is also used as a candidate genotype, and the standard coverage rate is calculated according to the reference set construction method of step 2, and the normal sample target genotype ratio vector NTi and the coverage rate of the cancer sample is recalculated. If this is still consistent with Tᵢⱼ<mean(NTᵢ)-3sd (NTᵢ), this locus is determined as an unstable microsatellite locus, otherwise it is only determined to be a potentially unstable microsatellite locus.

3. The multi-gene panel used in the examples also includes genes that are associated with colorectal cancer treatment and prognosis as clearly described in the NCCN treatment guidelines, and the genes that are associated with genetic susceptibility of colorectal cancer as clearly described in the NCCN genetic screening guidelines, and all exon detection of other gastrointestinal tumor related genes, wherein said multi-gene are as follows: BRAF, HRAS, KRAS, NRAS, PTCH1, APCBLM, BMPR1A, CHEK2, EpCAM, GREM1, MLH1, MSH2, MSH6, MUTYH, PMS2, POLD1, POLE, PTEN, SMAD4, STK11, TP53, AKT1, ATM, BRCA1, BRCA2, CDH1, EGFR, ERBB2, KIT, MET, PDGFRA, PIK3CA, SDHB, SDHC, SDHD.

### Detection and verification results

In the example, 65 IHC-positive tumor tissues and the corresponding paracancerous normal tissues were tested, and the correlation between the IHC results obtained from hospitals and the detection results of microsatellite instability and the MMR gene mutation was verified. The verification results are detailed in Tables 7 and 8. Based on the results, the following conclusions can be drawn:

Only a small fraction (44.6%) of IHC-positive patients can be detected to have the status of microsatellite instability. In other words, the current IHC detection developed in China is less sensitive to microsatellite status.

Among patients with IHC-positive and microsatellite instability, a total of 4 patients (13.8%) were detected to have germline MMR gene mutations identified as pathogenic or potentially pathogenic. They were diagnosed as Lynch syndrome, and were recommended to perform genetic testing for risk control. Another 7 patients (24.1%) were detected to have germline MMR gene mutations with unclear meaning, and further collection of family history and blood relative sequencing for diagnosis were necessary.

Of all the patients, 1 person detected APC potentially pathogenic germline mutation was a patient with FAP syndrome; 1 person detected CHEK2 potentially pathogenic germline mutation was a hereditary colorectal cancer patient; they were recommended to perform genetic testing for risk control; 2 persons detected ATM and MUTYH unknown germline mutations further need to collect family history and blood relative sequencing for diagnosis. This result illustrates the importance of simultaneously detecting multiple genetic susceptibility genes.

Of all the patients, 45 persons (67.7%) were detected mutations associated with treatment and prognosis as described in the treatment guideline. It provided a basis for subsequent treatment.

Further, we performed the gold standard test of traditional PCR detection on the above samples to verify the microsatellite stability detection results, in which the microsatellite stability detection is based on Next-generation sequencing method. Six single nucleotide repeats, NR-21, BAT-26, NR-27, BAT-25, NR-24 and MONO-27 and three loci, Penta C, PentaD and Amelogenin were complexly amplified using the gold standard test of PCR, and STR locus was detected on an ABI 3730x1 Genetic Analyzer. The PCR method finally determined 29 MSI samples and 36 MSS samples. Compared with the results of PCR analysis, it was found that the detection accuracy of the MSI of the present invention was 96.55% and the specificity was 100%. Only one of the samples was determined to be MSI-L according to the detection method of the present invention, and to be MSI-H according to the conventional method. After analysis, the tumor cells of this sample accounted for a relatively low proportion, thus affecting the determination result.

The PCR detection criteria are as follows in Table 2:

**Table 2: Markers and judging criteria for PCR gold standard testing**

| Marker category | Marker name | Repeat sequence | Description |
|---|---|---|---|
| Single nucleotide repeat markers | NR-21 | (A)₂₁ | Detect MSI |
| | NR-24 | (A)₂₄ | |
| | NR-27 | (A)₂₇ | |
| | BAT-25 | (A)₂₆ | |
| | BAT-26 | (A)₂₆ | |
| | MONO-27 | (A)₂₇ | |
| Pentanucleotide repeat markers | Penta C | (AAAAG)₃₋₁₅ | Identify potential sample mixture or contamination |
| | Penta D | (AAAAG)₂₋₁₇ | |
| Gender locus marker | Amel | | |
| *microsatellite stable(MSS): No change in single nucleotide repeat markers | | | |
| low-frequency MSI (MSI-L): Only one change in single nucleotide repeat markers | | | |
| high-frequency MSI, (MSI-H): ≥two changes in single nucleotide repeat markers | | | |

For the samples of the above mentioned numbers, the results of the PCR analysis are as follows:

**Table 3: PCR analysis results of the sample numbered RS1607586FFP**

| **Normal tissue** | | | **Diseased tissue** | | | |
|---|---|---|---|---|---|---|
| Marker | Fragment 1 | Fragment 2 | Marker | Fragment 1 | Fragment 2 | Fragment 3 |
| NR21 | 106.11 | | NR21 | 99.51 | 106.16 | |
| Bat26 | 178.44 | | Bat26 | 168.20 | 178.59 | |
| PentaC | 220.17 | 235.71 | PentaC | 219.99 | 235.67 | |
| NR27 | 86.02 | | NR27 | 73.99 | 79.49 | 86.06 |
| Bat25 | 119.51 | | Bat25 | 112.94 | 119.51 | |
| PentaD | 176.87 | | PentaD | 177.01 | | |
| Ame1 | 107.42 | 113.00 | Ame1 | 107.32 | 112.94 | |
| NR24 | 132.89 | | NR24 | 128.52 | 133.00 | |
| Mono27 | 172.03 | | Mono27 | 162.21 | 166.78 | 172.14 |

According to the NCI criteria for tumor MSI, the tumor tissue of this sample belongs to high microsatellite instability (MSI-H) type.

**Table 4: PCR analysis results of the sample numbered RS1608839FFP**

| **Normal tissue** | | | **Diseased tissue** | | |
|---|---|---|---|---|---|
| Marker | Fragment 1 | Fragment 2 | Marker | Fragment 1 | Fragment 2 |
| NR21 | 106.09 | | NR21 | 106.09 | |
| Bat26 | 177.46 | | Bat26 | 177.60 | |
| PentaC | 220.08 | 230.53 | PentaC | 220.07 | 230.60 |
| NR27 | 85.99 | | NR27 | 85.94 | |
| Bat25 | 119.50 | | Bat25 | 119.50 | |
| PentaD | 186.66 | 201.12 | PentaD | 186.78 | 201.27 |
| Ame1 | 107.43 | | Ame1 | 107.43 | |
| NR24 | 133.15 | | NR24 | 133.06 | |
| Mono27 | 173.02 | | Mono27 | 173.12 | |

According to the NCI criteria for tumor MSI, the tumor tissue of this sample belongs to microsatellite stability (MSS) type.

**Table 5: PCR analysis results of the sample numbered RS1611018FFP**

| **Normal tissue** | | | **Diseased tissue** | | | |
|---|---|---|---|---|---|---|
| Marker | Fragment 1 | Fragment 2 | Marker | Fragment 1 | Fragment 2 | Fragment 3 |
| NR21 | 105.92 | | NR21 | 100.38 | 106.03 | |
| Bat26 | 178.43 | | Bat26 | 167.10 | 178.43 | |
| PentaC | 230.41 | 235.67 | PentaC | 230.47 | 235.63 | 240.93 |
| NR27 | 85.65 | | NR27 | 75.88 | 81.36 | 85.77 |
| Bat25 | 119.50 | | Bat25 | 113.62 | 119.35 | |
| PentaD | 172.29 | 191.59 | PentaD | 172.29 | 191.43 | |
| Ame1 | 107.44 | 113.15 | Ame1 | 107.36 | 112.96 | |
| NR24 | 133.34 | | NR24 | 130.45 | 133.21 | |
| Mono27 | 172.29 | | Mono27 | 165.05 | 172.14 | |

According to the NCI criteria for tumor MSI, the tumor tissue of this sample belongs to high microsatellite instability (MSI-H) type.

**Table 6: PCR analysis results of the sample numbered RS1608823FFP**

| **Normal tissue** | | | **Diseased tissue** | | |
|---|---|---|---|---|---|
| Marker | Fragment 1 | Fragment 2 | Marker | Fragment 1 | Fragment 2 |
| NR21 | 106.92 | | NR21 | 107.04 | |
| Bat26 | 178.45 | | Bat26 | 178.60 | |
| PentaC | 220.18 | 230.57 | PentaC | 220.07 | 230.44 |
| NR27 | 86.96 | | NR27 | 87.00 | |
| Bat25 | 119.51 | | Bat25 | 119.50 | |
| PentaD | 186.62 | 191.50 | PentaD | 186.67 | 191.43 |
| Ame1 | 107.25 | 112.82 | Ame1 | 107.37 | 113.04 |
| NR24 | 133.06 | | NR24 | 133.17 | |
| Mono27 | 171.16 | | Mono27 | 171.26 | |

According to the NCI criteria for tumor MSI, the tumor tissue of this sample belongs to the microsatellite stability (MSS) type.

**Table 7: 65 samples of microsatellite NGS detection and PCR detection results**

| **Sample** | **IHC result** | | | | **MSI_NGS** | **MSI_PCR** | **MMR germline** | **Other germline mutation** | **MMR system** | **Drug-related mutation** |
|---|---|---|---|---|---|---|---|---|---|---|
| | **MLH1** | **MSH2** | **MSH6** | **PMS2** | | | | | | |
| RS1607578FFP | 0 | 1 | 1 | 0 | MSI-H | MSI-H | | | | BRAF_p.V600E, HRAS_amp |
| RS1607580FFP | 0 | 1 | 1 | 0 | MSS | MSS | | | | |
| RS1607582FFP | 1 | 1 | 2 | 0 | MSS | MSS | | | | |
| RS1607584FFP | 2 | 0 | 1 | 1 | MSI-H | MSI-H | MSH2_p.E878E | | MSH2_p.R711*+ MSH6_p.R1095H | KRAS_p.A146T |
| RS1607586FFP | 0 | 1 | 1 | 0 | MSI-H | MSI-H | | | MLH1_p.E519*+ MSH2_p.A230fs | KRAS_p.A59T |
| RS1608823FFP | 0 | 1 | 1 | 0 | MSS | MSS | | | | |
| RS1608826FFP | 0 | 1 | 1 | 0 | MSI-H | MSI-H | PMS2_p.E667Q | | | |
| RS1608827FFP | 0 | 2 | 2 | 1 | MSS | MSS | | | | KRAS_p.G12D |
| RS1608829FFP | 1 | 1 | 1 | 0 | MSS | MSS | | | | KRAS_p.G12V |
| RS1608831FFP | 0 | 1 | 1 | 0 | MSS | MSS | | | | KRAS_p.G12C |
| RS1608833FFP | 0 | 2 | 3 | 0 | MSI-H | MSI-H | | | | |
| RS1608837FFP | 0 | 3 | 3 | 0 | MSS | MSS | | | | |
| RS1608839FFP | 0 | 1 | 1 | 0 | MSS | MSS | | | PMS2_L42I | KRAS_p.G12V |
| RS1608841FFP | 0 | 1 | 1 | 0 | MSI-H | MSI-H | MSH6_p.R1095H | | MSH2_p.I884= | BRAF_p.V600E |
| RS1608843FFP | 0 | 1 | 1 | 0 | MSI-H | MSI-H | MSH6_splice | | | |
| RS1608845FFP | 0 | 1 | 1 | 0 | MSS | MSS | | | | NRAS_p.Q61R |
| RS1608847FFP | 0 | 1 | 1 | 0 | MSS | MSS | | ATM_p.P411L | | KRAS_p.G12V |
| RS1608849FFP | 0 | 1 | 1 | 0 | MSS | MSS | MLH1_p.T491N | | | KRAS_p.G12D |
| RS1608851FFP | 1 | 1 | 1 | 0 | MSS | MSS | | | | |
| RS1608853FFP | 1 | 0 | 0 | 1 | MSI-H | MSI-H | | | MSH2_p.T636= | KRAS_p.G13D |
| RS1609405FFP | 1 | 1 | 1 | 0 | MSS | MSS | | | | KRAS_p.G12D |
| RS1610952FFP | 1 | 1 | 1 | 0 | MSS | MSS | | | | BRAF_p.D594G |
| RS1610954FFP | 0 | 1 | 1 | 0 | MSS | MSS | | | | BRAF_p.V600E |
| RS1610956FFP | 1 | 1 | 1 | 0 | MSS | MSS | | | | |
| RS1610958FFP | 1 | 1 | 1 | 0 | MSS | MSS | MSH6_p.R58 _P59dup | | | |
| RS1610960FFP | 1 | 1 | 1 | 0 | MSS | MSS | | | | |
| RS1610962FFP | 0 | 1 | 1 | 0 | MSS | MSS | | APC_c.1409-2delA+APC_p.T15 56fs | PMS2_p.Q30P | HRAS_cn_amp+ KRAS_p.G12V |
| RS1610964FFP | 1 | 2 | 2 | 0 | MSS | MSS | | | | |
| RS1610966FFP | 2 | 3 | 2 | 0 | MSS | MSS | | | MLH1_p.C533Y | KRAS_p.G13D |
| RS1610968FFP | 1 | 1 | 1 | 0 | MSS | MSS | | | PMS2_p.R151H+PMS2_p.A127V+P MS2_p.L42I | |
| RS1610970FFP | 0 | 1 | 1 | 0 | MSI-H | MSI-H | MSH2_p.I735V | | MLH1_p.G67R | KRAS_p.G12D |
| RS1610972FFP | 1 | 3 | 2 | 0 | MSS | MSS | | | | KRAS_p.G12D |
| RS1610974FFP | 0 | 1 | 1 | 0 | MSS | MSS | | | PMS2_p.S118S | HRAS_cn_amp |
| RS1610976FFP | 0 | 1 | 1 | 0 | MSS | MSS | | | | KRAS_p.G12V |
| RS1610978FFP | 1 | 1 | 1 | 0 | MSS | MSS | | | | KRAS_p.G12D |
| RS1610980FFP | 0 | 1 | 1 | 0 | MSI-H | MSI-H | MLH1_p.R226Q | | MSH2_p.S233fs | KRAS_p.G12D |
| RS1610982FFP | 0 | 1 | 1 | 0 | MSI-H | MSI-H | | | MLHl_c.677+1G>A | |
| RS1610984FFP | 0 | 2 | 1 | 0 | MSS | MSS | | | | KRAS_p.G12A |
| RS1610986FFP | 0 | 2 | 3 | 0 | MSI-H | MSI-H | | | PMS2_p.S118S+PMS2_p.I144fs | KRAS_p.Q129fs |
| RS1610988FFP | 1 | 1 | 1 | 0 | MSI-H | MSI-H | PMS2_p.S358F | | MSH6_p.R361H+PMS2_p.M1? | KRAS_p.K117R |
| RS1610990FFP | 1 | 0 | 0 | 1 | MSI-H | MSI-H | | CHEK2_P.H414Y | MSH2_p.K393^{*}MSH2_p.L730^{*} | |
| RS1610992FFP | 0 | 0 | 2 | 0 | MSI-H | MSI-H | | | | BRAF_p.V600E +HRAS_p.E31K |
| RS1610994FFP | 0 | 2 | 3 | 0 | MSI-H | MSI-H | MLH1_c.677+1G >A | | MSH6_p.A36T+MSH6_p.A40fs+MLH1_p.K618del | KRAS_p.G13D |
| RS1610996FFP | 1 | 0 | 1 | 1 | MSS | MSS | | | PMS2_p.S118S | HRAS_cn_amp |
| RS1610998FFP | 1 | 0 | 0 | 1 | MSI-H | MSI-H | | | MSH6_p.R554C | KRAS_p.V14I |
| RS1611000FFP | 0 | 2 | 3 | 0 | MSI-H | MSI-H | | | | BRAF_p.V600E +HRAS_p.A18V |
| RS1611002FFP | 1 | 0 | 3 | 2 | MSS | MSS | | | | |
| RS1611004FFP | 0 | 1 | 1 | 0 | MSI-H | MSI-H | | | | |
| RS1611006FFP | 0 | 2 | 3 | 0 | MSI-H | MSI-H | | | | |
| RS1611008FFP | 1 | 0 | 2 | 1 | MSS | MSS | | | | KRAS_p.G12D |
| RS1611010FFP | 2 | 0 | 0 | 2 | MSI-L | MSI-H | | | | KRAS_p.Q61R |
| RS1611012FFP | 1 | 0 | 0 | 1 | MSI-H | MSI-H | | | PMS2_p.A49V | KRAS_p.Q61R |
| RS1611014FFP | 1 | 2 | 2 | 0 | MSS | MSS | | | | KRAS_p.G12D |
| RS1611016FFP | 0 | 3 | 3 | 0 | MSS | MSS | | | | |
| RS1611018FFP | 0 | 1 | 1 | 0 | MSI-H | MSI-H | MSH2_p.L387F | | MLH1_p.R487*+ MLH1_p.F530fs | KRAS_p.G13D |
| RS1611020FFP | 0 | 1 | 1 | 0 | MSI-H | MSI-H | | | | BRAF_p.V600E |
| RS1611022FFP | 0 | 1 | 1 | 0 | MSS | MSS | PMS2_p.M362K | | | KRAS_p.G12D |
| RS1611024FFP | 0 | 1 | 1 | 0 | MSS | MSS | | | | KRAS_p.G12VH RAS_cn_amp |
| RS1611026FFP | 0 | 1 | 1 | 0 | MSI-H | MSI-H | MET_p.R591W+MLH1_p.E609fs | | MLH1_p.Q60P | HRAS_cn_amp |
| RS1611028FFP | 1 | 1 | 1 | 0 | MSI-H | MSI-H | | | PMS2_p.E109K+PMS2_p.M312fs | |
| RS1611032FFP | 1 | 3 | 3 | 0 | MSS | MSS | | | | KRAS_p.G12C |
| RS1611034FFP | 0 | 3 | 3 | 0 | MSI-H | MSI-H | | | MSH6_p.A16V | BRAF_p.V600E |
| RS1611036FFP | 1 | 3 | 3 | 0 | MSS | MSS | | | | KRAS_p.A146T |
| RS1611038FFP | 0 | 1 | 1 | 0 | MSI-H | MSI-H | | MUTYH_p.Y453C | MSH2_p.R243W | |
| RS1611040FFP | 0 | 0 | 0 | 0 | MSI-H | MSI-H | MSH2_p.P125del | | MSH2_p.K423fs | KRAS_p.A59T |

| | | | | | | | | | | |
|---|---|---|---|---|---|---|---|---|---|---|
| Remarks: IHC results indicate: 0=IHC no expression; 1=IHC display+; 2=IHC display++; 3=IHC display+++ Germline mutation pathogenicity: pathogenic or potentially uncertain | | | | | | | | | | |

In addition, the inventors analyze the distribution of 22 microsatellite loci in 65 samples, each of which satisfies the following conditions: the number of the sequencing fragments' read covering the target genotype corresponding to the microsatellite loci exceeds 10.The distribution of each microsatellite locus in 65 samples, and the judgment results of the stability state of each microsatellite locus in each sample are detailed in Table 8 below. NA indicates no or no condition.

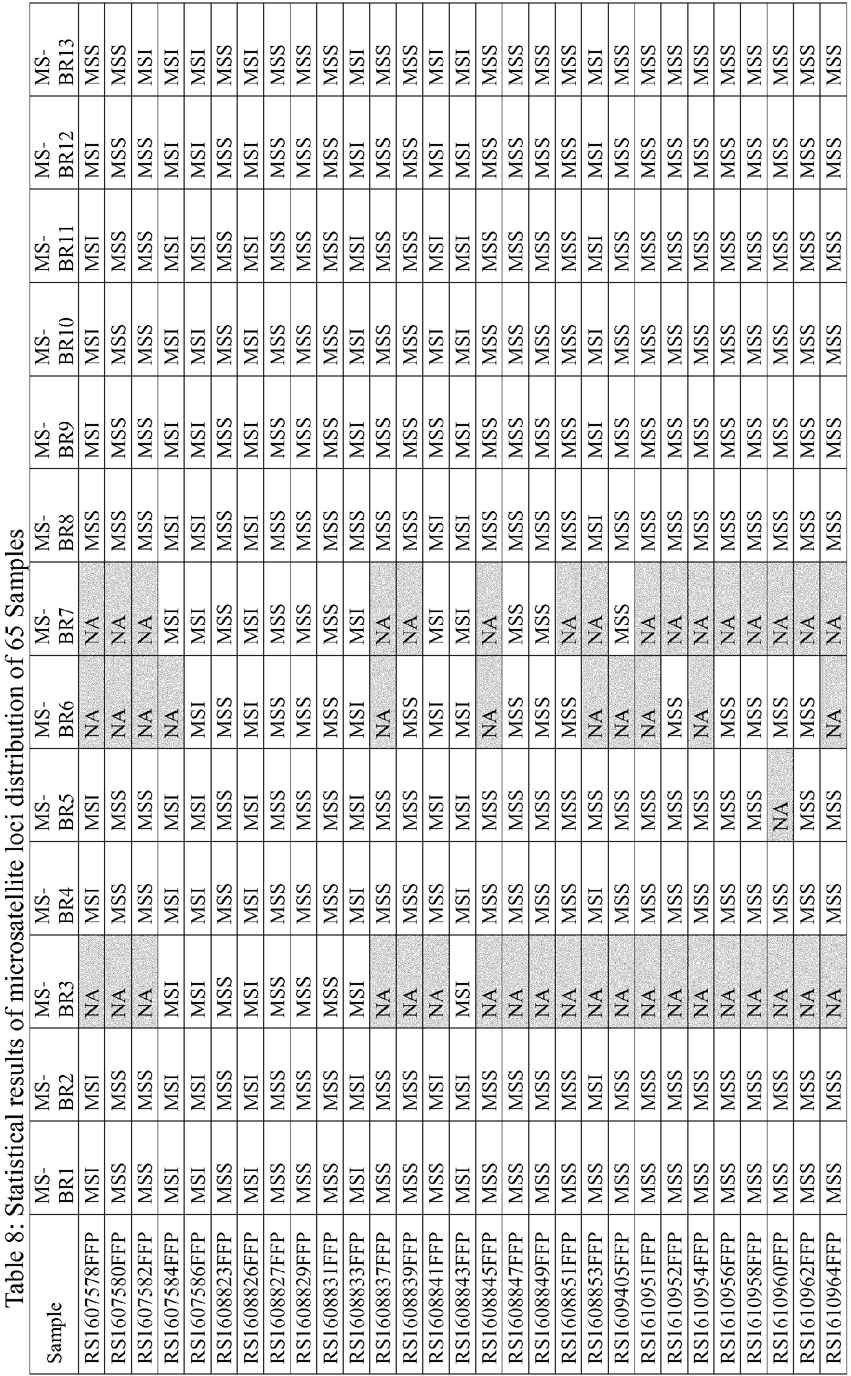

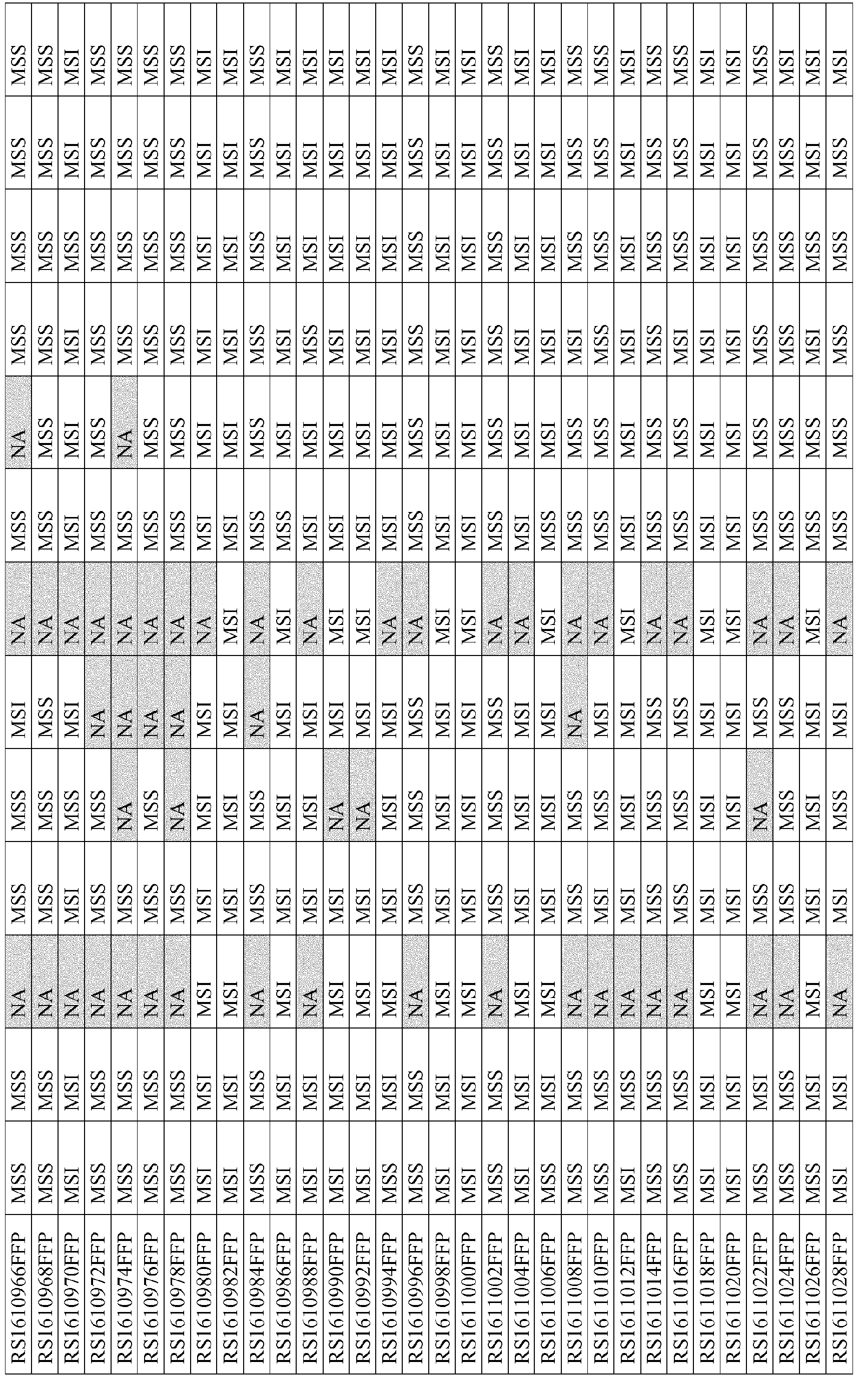

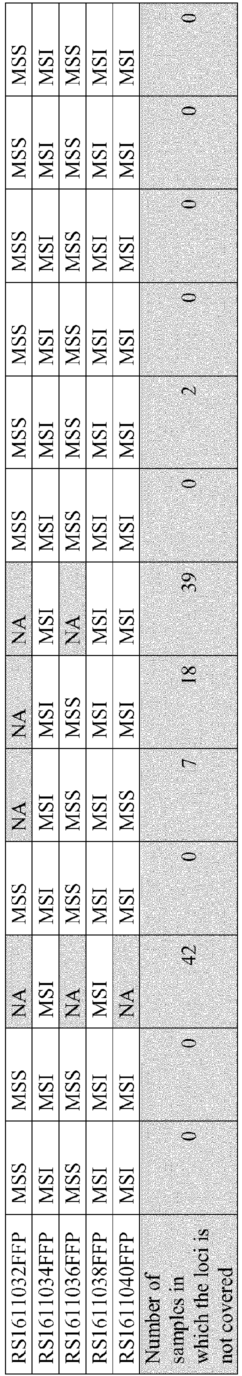

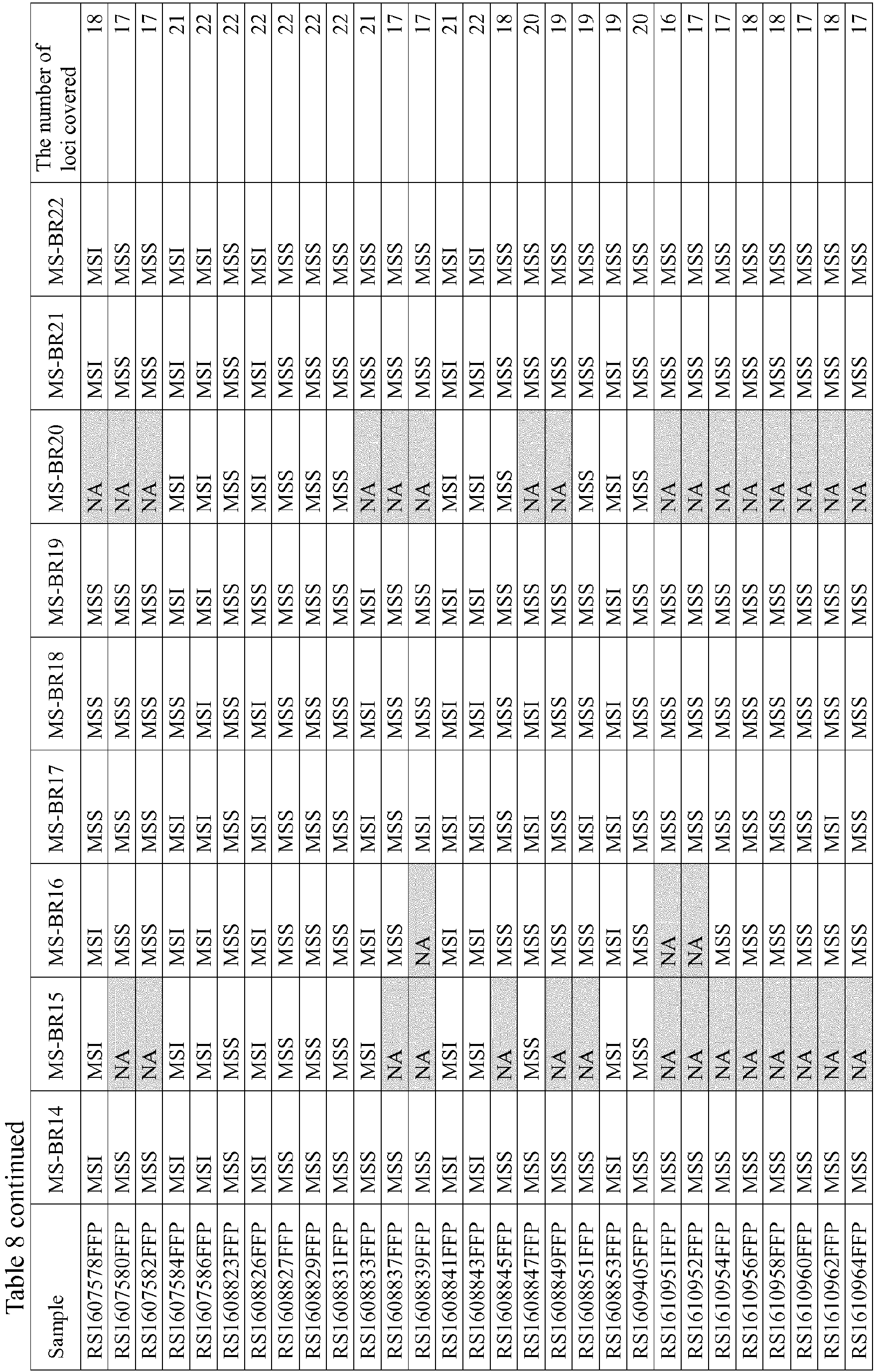

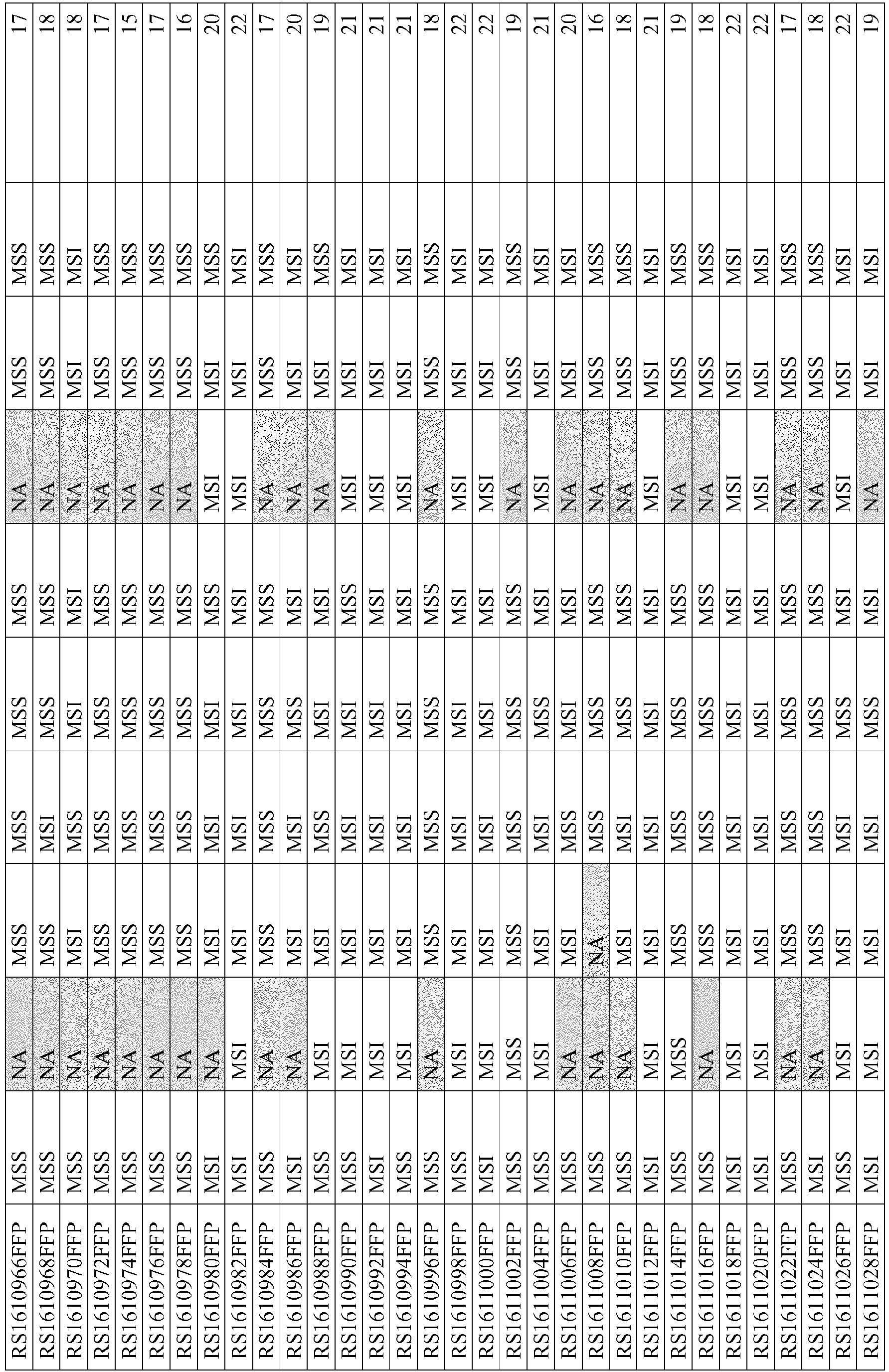

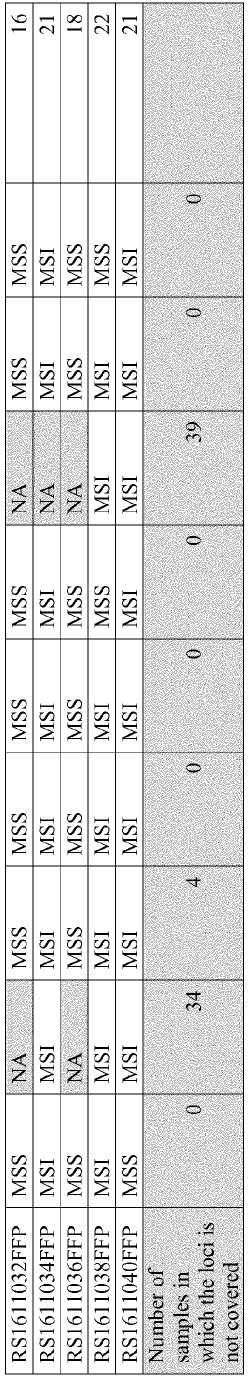

## Claims

1. A biomarker panel comprising 22 microsatellite loci as shown in Table 1, or comprising a combination of 15, 16, 17, 18, 19, 20, or 21 of loci as shown in Table 1.

2. The biomarker panel of claim 1, wherein said panel comprises 22 microsatellite loci as shown in Table 1.

3. A biomarker panel comprising a combination of microsatellite loci and one or more genes, wherein the microsatellite loci include 22 microsatellite loci as shown in Table 1 or a combination of 15, 16, 17, 18, 19, 20, or 21 of microsatellite loci as shown in Table 1, and wherein the one or more genes are selected form the following 36 genes: BRAF, HRAS, KRAS, NRAS, PTCH1, APCBLM, BMPR1A, CHEK2, EpCAM, GREM1, MLH1, MSH2, MSH6, MUTYH, PMS2, POLD1, POLE, PTEN, SMAD4, STK11, TP53, AKT1, ATM, BRCA1, BRCA2, CDH1, EGFR, ERBB2, KIT, MET, PDGFRA, PIK3CA, SDHB, SDHC, and SDHD.

4. The biomarker panel of claim 3, wherein said panel comprises 22 microsatellite loci as shown in Table 1, and/or the 36 genes.

5. A kit for genetic detection for prognostic evaluation and/or treatment regimen selection for stage II colorectal cancer, and for diagnosis, prognosis assessment, and/or treatment regimen selection of Lynch syndrome (HNPCC), **characterized in that** said kit comprises a detection reagent for detecting the biomarker panel according to claim 1 or 2.

6. A kit for simultaneously performing genetic detections to assess prognosis and/or select treatment regimen for colorectal cancer, assess genetic susceptibility to colorectal cancer, and assess genetic susceptibility to gastrointestinal cancer, **characterized in that** said kit comprises a detection reagent detecting the biomarker panel according to claim 3 or 4.

7. The kit of claim 6, wherein the genetic detections comprise the genetic detections of familial adenomatous polyposis, sporadic CRC, Lynch syndrome, and/or sporadic MSI+CRC.

8. The kit of any of claims 5-7, wherein the reagent is a reagent for performing Next-generation sequencing (NGS).

9. Use of a reagent detecting the biomarker panel of any of claims 1-4 in the preparation of a kit, wherein the kit is used for genetic detections to assess prognosis and/or select treatment regimen for colorectal cancer, assess genetic susceptibility to colorectal cancer, and assess genetic susceptibility to gastrointestinal cancer.

10. The use of claim 9, wherein the reagent is a reagent for performing Next-generation sequencing (NGS).

11. The use of claim 9, wherein the genetic detection for the colorectal cancer prognosis assessment and/or treatment regimen selection is a genetic detection for Lynch syndrome (HNPCC) prognosis assessment and/or treatment regimen selection.

12. A method for determining candidate microsatellite loci in a genetic detection capable of being used for colorectal cancer prognosis assessment and/or treatment regimen selection based on Next-generation sequencing (NGS), comprising the steps of:
(1) simultaneous multi-gene targeted capture detection of multiple microsatellite loci in multiple normal tissue samples based on Next-generation sequencing;
(2) For any one of the microsatellite loci ***i*,** the number of sequencing fragments' reads covering different lengths of target genotypes corresponding to the loci are counted based on the NGS data, and the target genotype is the genotype of the microsatellite locus in normal tissue samples;
(3)calculating the coverage rate of the target genotypes corresponding to the microsatellite loci according to the number of the sequencing fragments' read and constructing a standard length distribution reference set of the microsatellite loci, thereby calculating the average coverage mean(NTᵢ) and standard deviation sd(NTᵢ) of one or two length types covering the most, wherein if the number of reads of sequencing fragments covering the second largest number is less than 75% of that of the length types covering the most, only the length types covering the most is considered; and calculating the average coverage and standard deviation; if the number of reads of sequencing fragments covering the second largest number is greater than 75% of that of the length types covering the most, the two types of lengths that cover the most are considered, with the average at this time being the sum of the average coverage of the two length types;
(4) for colorectal cancer samples, the coverage rate of the target genotype determined is also calculated according to the above steps; if the coverage rate is <mean(NTᵢ)-3sd(NTᵢ), the microsatellite locus is a unstable microsatellite locus, which is determined to be a candidate microsatellite locus in a genetic detection, wherein said genetic detection can be used for prognosis assessment and/or treatment regimen selection of colorectal cancer.

13. The microsatellite loci determined by the method of claim 12, comprising 22 microsatellite loci as shown in Table 1.

14. The method of claim 12, wherein the genetic detection for the colorectal cancer prognosis assessment and/or treatment regimen selection is one for Lynch syndrome (HNPCC) prognostic assessment and/or treatment regimen selection.

15. A method for determining a stability state of a microsatellite locus in a colorectal cancer sample based on Next-generation sequencing, comprising the steps of:
(1) simultaneously performing a multi-gene targeted capture detection of a plurality of microsatellite loci in the sample based on Next-generation sequencing, the plurality of microsatellite loci comprising 22 microsatellite loci as shown in Table 1 or a combination of 15, 16, 17, 18, 19, 20, 21 of loci as shown in Table 1;
(2) among the plurality of microsatellite loci, the number of microsatellite loci in which the number of the sequencing fragments' read covering the target genotype corresponding to the microsatellite loci exceeds 10 is n, and the target genotype is the genotype of microsatellite loci in normal tissue samples, n≥15; for any microsatellite locus in n, it can be determined by the above method whether the coverage rate Tᵢⱼ< mean (NTᵢ)-3sd (NTᵢ) is satisfied; when the microsatellite loci are 22 microsatellite loci in Table 1, they can be directly calculated according to the mean (NTᵢ) and sd (NTᵢ) of Table 1;
(3) for the plurality of microsatellite loci, if the number of unstable microsatellite loci is >40%*n, the sample is determined to be a high MSI sample; if the number is 15%-40 %*n, the sample is determined to be a low MSI sample; if the number of samples is <15%*n, the sample is determined to be an MSS.

16. The method of claim 15, wherein the plurality of microsatellite loci are 22 microsatellite loci as shown in Table 1.

17. A method for simultaneously determining the stability state of multiple microsatellite loci and detecting disease-related genes in a patient's colorectal cancer sample, wherein said method provides clinical guidance for the risk control, treatment regimen and/or prognosis assessment for the colorectal cancer of the patient or family based on Next-generation sequencing, comprising the steps of:
(1) simultaneously detecting the plurality of microsatellite loci of claim 15 and any one or more of the following 36 genes: BRAF, HRAS, KRAS, NRAS, PTCH1, APCBLM, BMPR1A, CHEK2, EpCAM, GREM1, MLH1, MSH2, MSH6, MUTYH, PMS2, POLD1, POLE, PTEN, SMAD4, STK11, TP53, AKT1, ATM, BRCA1, BRCA2, CDH1, EGFR, ERBB2, KIT, MET, PDGFRA, PIK3CA, SDHB, SDHC, SDHD;
(2) determining the stability state of a microsatellite locus of the sample according to the method of claim 15;
(3) obtaining the detection result of the one or more disease-related genes according to the sequencing results;
(4) providing clinical guidance to the risk control, treatment regimen and/or prognosis assessment for the colorectal cancer of the patient or family in combination with the results of the steps (2) and (3).

18. A kit for the method of any of claims 12-17, comprising reagents detecting the multiple microsatellite loci.
